(19) Europäisches Patentamt
European Patent Office
Office européen des brevets

(11) EP 2 768 493 B1

(12) **EUROPEAN PATENT SPECIFICATION**

(45) Date of publication and mention of the grant of the patent:
**31.07.2019 Bulletin 2019/31**

(21) Application number: **12780523.2**

(22) Date of filing: **17.10.2012**

(51) Int Cl.:
***A61K 31/05*** (2006.01) ***A61K 31/352*** (2006.01)
***A61P 35/00*** (2006.01)

(86) International application number:
**PCT/GB2012/052565**

(87) International publication number:
**WO 2013/057487 (25.04.2013 Gazette 2013/17)**

(54) **PHYTOCANNABINOIDS FOR USE IN THE TREATMENT OF BREAST CANCER**

PFLANZLICHE CANNABINOIDE ZUR VERWENDUNG BEI DER BEHANDLUNG VON BRUSTKREBS

PHYTOCANNABINOÏDES DESTINÉS À ÊTRE UTILISÉS DANS LE TRAITEMENT DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2011 GB 201117956**
**21.10.2011 US 201161550069 P**

(43) Date of publication of application:
**27.08.2014 Bulletin 2014/35**

(73) Proprietor: **GW Pharma Limited**
**Salisbury, Wiltshire SP4 0jQ (GB)**

(72) Inventors:
- **SANCHEZ, Cristina**
  **E-28040 Madrid (ES)**
- **GUZMAN, Manuel**
  **E-28040 Madrid (ES)**
- **WRIGHT, Stephen**
  **Salisbury SP4 0JQ (GB)**
- **STOTT, Colin**
  **Salisbury SP4 0JQ (GB)**
- **MUNOZ CAFFAREL, Maria**
  **E-28040 Madrid (ES)**
- **ANDRADAS, Clara**
  **E-28040 Madrid (ES)**
- **PEREZ GOMEZ, Eduardo**
  **E-28040 Madrid (ES)**

(74) Representative: **HGF Limited**
**4th Floor**
**Merchant Exchange**
**17-19 Whitworth Street West**
**Manchester M1 5WG (GB)**

(56) References cited:
**WO-A1-2008/144475**

- **CAFFAREL MARÍA M ET AL: "Cannabinoids reduce ErbB2-driven breast cancer progression through Akt inhibition", MOLECULAR CANCER, BIOMED CENTRAL, LONDON, GB, vol. 9, no. 1, 22 July 2010 (2010-07-22), page 196, XP021077934, ISSN: 1476-4598, DOI: 10.1186/1476-4598-9-196 cited in the application**
- **M. M. CAFFAREL: "9-Tetrahydrocannabinol Inhibits Cell Cycle Progression in Human Breast Cancer Cells through Cdc2 Regulation", CANCER RESEARCH, vol. 66, no. 13, 1 July 2006 (2006-07-01), pages 6615-6621, XP055048739, ISSN: 0008-5472, DOI: 10.1158/0008-5472.CAN-05-4566**
- **TAKEDA ET AL: "DELTA<9>-Tetrahydrocannabinol enhances MCF-7 cell proliferation via cannabinoid receptor-independent signaling", TOXICOLOGY, LIMERICK, IR, vol. 245, no. 1-2, 28 December 2007 (2007-12-28), pages 141-146, XP022492672, ISSN: 0300-483X, DOI: 10.1016/J.TOX.2007.12.019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **THOMAS A ET AL: "Cannabidiol displays unexpectedly high potency as an antagonist of CB1 and CB2 receptor agonists in vitro", BRITISH JOURNAL OF PHARMACOLOGY,, vol. 150, 1 March 2007 (2007-03-01), pages 613-623, XP002446113, ISSN: 0007-1188, DOI: 10.1038/SJ.BJP.0707133**
- **R G Pertwee: "The diverse CB1 and CB2 receptor pharmacology of three plant cannabinoids: Delta(9)-tetrahydrocannabinol, cannabidiol and Delta(9)-tetrahydrocannabivarin", BRITISH JOURNAL OF PHARMACOLOGY, vol. 153, no. 2, 1 January 2008 (2008-01-01), pages 199-215, XP055125148, ISSN: 0007-1188, DOI: 10.1038/sj.bjp.0707442**

## Description

The present invention relates to the phytocannabinoid cannabidiol (CBD) for use in the treatment of aggressive breast cancer characterised by overexpression of the Her2 gene.

## BACKGROUND TO THE INVENTION

Breast cancer occurs due to mutations in the genes responsible for regulating the growth of breast cells which causes the cells to grow in an unregulated manner. Usually breast cancer either begins in the cells of the milk producing glands, known as the lobules, or in the ducts. Less commonly breast cancer can begin in the stromal tissues. These include the fatty and fibrous connective tissues of the breast.

Over time the breast cancer cells can invade nearby tissues such the underarm lymph nodes or the lungs in a process known as metastasis.

The stage of the breast cancer, the size of the tumour and it's rate of growth are all factors which determine the type of treatment that is offered. Treatment options include surgery to remove the tumour, drug treatment which includes chemotherapy and hormonal therapy, radiation therapy and immunotherapy.

The prognosis and survival rate varies widely; the five year relative survival rates vary from 98% to 23% depending on the type of breast cancer that occurs. Worldwide breast cancer constitutes 23% of all cancers with the majority of breast cancers occurring in women.

A particularly aggressive form of breast cancer is characterised by amplification of the Her2 gene which results in overexpression of its protein product. Her2 stands for "human epidermal growth factor receptor 2" and is also known as ErbB-2.

Approximately 15 to 20% of breast cancers have amplification of the Her2 gene. This receptor is stimulated by a growth factor which causes the cell to divide; in the absence of the growth factor the cell will normally stop growing. Overexpression of this receptor in breast cancer is associated with increased resistance of the breast tumour to treatment and recurrence of the disease.

The compound trastuzumab is a monoclonal antibody to Her2 and has improved the 5 year relative survival rates of stage 1 to 3 Her2-positive breast cancers to approximately 87%, however trastuzumab is very expensive, a full course costs approximately $70,000, in addition approximately 2% of patients suffer significant heart damage from its use.

Although the use of this therapy has clearly improved the outcome of patients with Her2-positive tumours, innate and acquired resistance to trastuzumab is still a clinical challenge. Indeed only 25% of Her2-positive tumours respond to trastuzumab and most of the responders eventually relapse.

Cannabinoids have been shown to have an anti-proliferative effect on different cancer cell lines. The cannabinoids THC, THCA, CBD, CBDA, CBG and CBC and the cannabinoid BDS THC and CBD were tested on eight different cell lines including DU-145 (hormone-sensitive prostate cancer), MDA-MB-231 (breast cancer), CaCo-2 (colorectal cancer) and C6 (glioma cells). Furthermore as well as pure cannabinoids a THC botanical drug substance (BDS) and a CBD BDS containing about 95% (w/w) of the respective primary cannabinoid were used, (Ligresti, 2006).

CBD was also shown to inhibit id-1 gene expression in some aggressive forms of breast cancer. The cell lines used were MDA-MB231 and MDA-MB436, (McAllister *et al.* 2007).

In the application WO 2008/144475, McAllister also describes the use of a combination of the cannabinoids THC and CBD in the treatment glioma. Furthermore CBD is described for use in the treatment of breast cancer as exemplified by the cell lines MDA-MB231 and MDA-MB436, but not cell lines characterized by overexpression of the Her2 gene. In glioma the combination consisted of a high ratio of THC to a low ratio of CBD (4:1 THC:CBD). The problem with such a composition is that the high ratio of THC leads to side effects such as psychosis and anxiety. This work is further described in McAllister *et al.* 2010.

The present applicants in application WO 2009/147439 themselves describe the use of a combination of cannabinoids, particularly tetrahydrocannabinol (THC) and cannabidiol (CBD), in the treatment of cancer. In particular the cancer to be treated is a brain tumour, more particularly a glioma; more particularly still a glioblastoma multiforme (GBM).

Caffarel *et al.* (2006, 2008) describes the mechanism whereby THC inhibits cell cycle proliferation and describes the use of THC in an animal model of breast cancer.

More recently Caffarel *et al.* 2010 documented the anti-tumour action of THC in different models of breast cancer, including cell cultures, xenografted mice, and more recently a genetically engineered mouse model of ErbB2-positive breast cancer, the THC was injected peritumourally. The paper describes that CB2 agonists such as THC and JWH-133 were able to reduce tumour growth and number in ErbB2-driven breast cancer. The paper additionally describes that 91% of ErbB2-positive tumours express the CB2 receptor.

Shrivastava (2011) describes the how cannabidiol induces programmed cell death in breast cancer cells by co-ordinating cross-talk between apoptosis and autophagy.

Because breast cancer is such a major problem, and existing treatments have limited long-term success, it is

a primary object of the present invention to identify alternative treatments which might improve a patient's prognosis.

It is an object to provide novel compounds which may be used in the treatment of aggressive breast cancer characterised by overexpression of the Her2 gene since only about 25% of patients with this subset of breast cancer respond to the existing drug of choice trastuzumab and of those treated many relapse and die from secondary tumours which have metastasised from the primary cancer forming secondary tumours in the lung and lymph nodes.

**BRIEF SUMMARY OF THE DISCLOSURE**

In accordance with a first aspect of the present invention there is provided Cannabidiol (CBD) for use in the treatment of aggressive breast cancer characterised by overexpression of the Her2 gene.

Preferably the CBD is in the form of a botanical drug substance (BDS).

The CBD is present in a therapeutically acceptable amount, which may, for example, be between 1mg and 2000mg.

The human dose equivalent (HED) can be estimated using the following formula:

$$\text{HED} = \text{Animal dose (mg/kg)}\ \textit{multiplied by}\ \frac{\text{Animal } K_m}{\text{Human } K_m}$$

The $K_m$ for a mouse is 3 and the $K_m$ for a human is 37.

The CBD may further comprise a non-cannabinoid chemotherapeutic agent.

The non-cannabinoid chemotherapeutic agent may be a monoclonal antibody, such as trastuzumab.

In this specification the following terms are used and are intended to have the following meanings / definitions: "Cannabinoids" are a group of compounds including the endocannabinoids, the phytocannabinoids and those which are neither endocannabinoids or phytocannabinoids, hereafter "syntho-cannabinoids".

"Endocannabinoids" are endogenous cannabinoids, which are high affinity ligands of CB1 and CB2 receptors.

"Phytocannabinoids" are cannabinoids that originate in nature and can be found in the cannabis plant. The phytocannabinoids can be present in an extract including a botanical drug substance, isolated, or reproduced synthetically.

"Syntho-cannabinoids" are those compounds capable of interacting with the cannabinoid receptors (CB1 and / or CB2) but are not found endogenously or in the cannabis plant. Examples include WIN 55212 and rimonabant.

An "isolated phytocannabinoid" is one which has been extracted from the cannabis plant and purified to such an extent that all the additional components such as secondary and minor cannabinoids and the non-cannabinoid fraction have been removed.

A "synthetic cannabinoid" is one which has been produced by chemical synthesis this term includes modifying an isolated phytocannabinoid, by for example forming a pharmaceutically acceptable salt thereof.

A "botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug derived from one or more plants, algae, or microscopic fungi. It is prepared from botanical raw materials by one or more of the following processes: pulverisation, decoction, expression, aqueous extraction, ethanolic extraction or other similar processes." A botanical drug substance does not include a highly purified or chemically modified substance derived from natural sources. Thus, in the case of cannabis, BDS derived from cannabis plants do not include highly purified Pharmacopoeial grade cannabinoids.

In the present invention a BDS is considered to have two components: the phytocannabinoid-containing component and the non-phytocannabinoid containing component. Preferably the phytocannabinoid-containing component is the larger component comprising greater than 50% (w/w) of the total BDS and the non-phytocannabinoid containing component is the smaller component comprising less than 50% (w/w) of the total BDS.

The amount of phytocannabinoid-containing component in the BDS may be greater than 55%, through 60%, 65%, 70%, 75%, 80% to 85% or more of the total extract. The actual amount is likely to depend on the starting material used and the method of extraction used.

The "principle phytocannabinoid" in a BDS is the phytocannabinoid that is present in an amount that is higher than that of the other phytocannabinoids. Preferably the principle phytocannabinoid is present in an amount greater than 40% (w/w) of the total extract. More preferably the principle phytocannabinoid is present in an amount greater than 50% (w/w) of the total extract. More preferably still the principle phytocannabinoid is present in an amount greater than 60% (w/w) of the total extract.

The amount of the principle phytocannabinoid in the BDS is preferably greater than 50% of the phytocannabinoid-containing fraction, more preferably still greater than 55% of the phytocannabinoid-containing fraction, and more preferably still greater than 60% through 65%, 70%, 75%, 80%, 85%, 90% and 95% of the phytocannabinoid-containing fraction.

The "secondary phytocannabinoid/s" in a BDS is the phytocannabinoid/s that is / are present in significant proportions. Preferably the secondary phytocannabinoid is present in an amount greater than 5% (w/w) of the total extract, more preferably greater than 10% (w/w) of the total extract, more preferably still greater than 15% (w/w) of the total extract. Some BDS's will have two or more secondary phytocannabinoids that are present in significant amounts. However not all BDS's will have a secondary phytocannabinoid.

The "minor phytocannabinoid/s" in a BDS can be described as the remainder of all the phytocannabinoid components once the principle and secondary phytocannabinoids are accounted for. Preferably the minor phytocannabinoids are present in total in an amount of less than 5% (w/w) of the total extract, and most preferably the minor phytocannabinoid is present in an amount less than 2% (w/w) of the total extract.

The term "consisting essentially of" is limited to the phytocannabinoids which are specified, it does not exclude non-cannabinoid components that may also be present.

Typically the non-phytocannabinoid containing component of the BDS comprises terpenes, sterols, triglycerides, alkanes, squalenes, tocopherols and carotenoids.

These compounds may play an important role in the pharmacology of the BDS either alone or in combination with the phytocannabinoid.

The "terpene fraction" may be of significance and can be broken down by the type of terpene: monoterpene or sesquiterpene. These terpene components can be further defined in a similar manner to the cannabinoids.

The amount of non-phytocannabinoid containing component in the BDS may be less than 45%, through 40%, 35%, 30%, 25%, 20% to 15% or less of the total extract. The actual amount is likely to depend on the starting material used and the method of extraction used.

The "principle monoterpene/s" in a BDS is the monoterpene that is present in an amount that is higher than that of the other monoterpenes. Preferably the principle monoterpene/s is present in an amount greater than 20% (w/w) of the total terpene content. More preferably the principle monoterpene is present in an amount greater than 30% (w/w) of the total terpene content, more preferably still greater than 40% (w/w) of the total terpene content, and more preferably still greater than 50% (w/w) of the total terpene content. The principle monoterpene is preferably a myrcene or pinene. In some cases there may be two principle monoterpenes. Where this is the case the principle monoterpenes are preferably a pinene and / or a myrcene.

The "principle sesquiterpene" in a BDS is the sesquiterpene that is present in an amount that is higher than all the other sesquiterpenes. Preferably the principle sesquiterpene is present in an amount greater than 20% (w/w) of the total terpene content, more preferably still greater than 30% (w/w) of the total terpene content. The principle sesquiterpene is preferably a caryophyllene and / or a humulene.

The sesquiterpene components may have a "secondary sesquiterpene". The secondary sesquiterpene is preferably a pinene, which is preferably present at an amount greater than 5% (w/w) of the total terpene content, more preferably the secondary sesquiterpene is present at an amount greater than 10% (w/w) of the total terpene content.

The secondary sesquiterpene is preferably a humulene which is preferably present at an amount greater than 5% (w/w) of the total terpene content, more preferably the secondary sesquiterpene is present at an amount greater than 10% (w/w) of the total terpene content.

Alternatively botanical extracts may be prepared by introducing isolated phytocannabinoids or their synthetic equivalent into a non-cannabinoid plant fraction as can be obtained from a zero cannabinoid plant or one or more non-cannabinoid components found in the cannabis plant such as terpenes.

The structures of the phytocannabinoids CBD and THC are as shown below:

| | | |
|---|---|---|
| CBD | Cannabidiol | 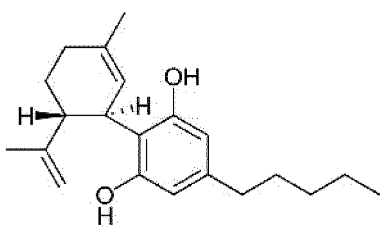 |

(continued)

| | | |
|---|---|---|
| THC | Tetrahydrocannabinol | 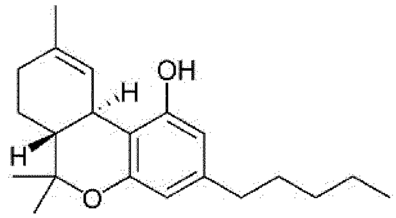 |

Phytocannabinoids can be found as either the neutral (decarboxylated form) or the carboxylic acid form depending on the method used to extract the cannabinoids. For example it is known that heating the carboxylic acid form will cause most of the carboxylic acid form to decarboxylate into the neutral form.

Where a synthetic phytocannabinoid is used the term is intended to include compounds, metabolites or derivatives thereof, and pharmaceutically acceptable salts of such compounds.

The term "pharmaceutically acceptable salts" refers to salts or esters prepared from pharmaceutically acceptable non-toxic bases or acids, including inorganic bases or acids and organic bases or acids, as would be well known to persons skilled in the art. Many suitable inorganic and organic bases are known in the art.

For the purpose of this invention the term 'treatment' is intended to encompass decreasing the viability of cancer cells and their ability to metastasise, and a therapeutically effective amount is an amount that achieves this aim.

**BRIEF DESCRIPTION OF THE DRAWINGS**

Embodiments of the invention are further described hereinafter with reference to the accompanying drawings, in which

Figure 1 shows that human Her2-positive breast cancer cell lines are sensitive to THC;

Figure 2 shows human Her2-positive breast cancer cell lines are sensitive to THC and CBD;

Figure 3 shows the combination of submaximal concentrations of THC and CBD enhances breast cancer cell death;

Figure 4 shows the combination of THC and CBD decreases breast cancer cell viability in a synergistic manner;

Figure 5 shows that human Her2-positive breast cancer cells are sensitive to THC *in vivo;*

Figure 6 shows that human BT474 cells are sensitive to phytocannabinoids *in vivo;*

Figure 7 shows that phytocannabinoids improve trastuzumab anti-tumour action *in vivo;*

Figure 8 shows that human Her2-positive breast cancer cells are sensitive to different THC:CBD ratios;

Figure 9 shows highly metastatic Her2-positive breast cancer cells are sensitive to THC and CBD;

Figure 10 shows trastuzumab resistant Her2-positive breast cancer cell lines are sensitive to THC and CBD;

Figure 11 shows the combination of THC and CBD in ratios up to 1:9 reduces triple-negative breast cancer cell viability;

Figure 12 shows THC and CBD reduce triple-negative breast cancer cell viability in an additive manner;

Figure 13 shows the combination of THC and CBD in a 1:9 ratio significantly reduces Her2-overexpressing breast cancer cell viability;

Figure 14 shows THC and CBD reduce Her2-overexpressing breast cancer cell viability in an additive manner;

Figure 15 shows the combination of THC and CBD in a 1:9 ratio reduces Her2-overexpressing trastuzumab-resistant breast cancer cell viability;

Figure 16 shows THC and CBD reduce Her2-overexpressing trastuzumab-resistant breast cancer cell viability in an additive manner;

Figure 17 shows the combination of THC and CBD in a 1:9 ratio reduces Her2-overexpressing highly metastatic breast cancer cell viability;

Figure 18 shows THC and CBD reduce Her2-overexpressing highly metastatic breast cancer cell viability in an additive manner; and

Figure 19 shows human Her2-positive breast cancer cells are sensitive to 1:9 THC:CBD combinations *in vivo.*

Legend to Figures

Figure 1: (A) Her2 and (B) $CB_1$ and $CB_2$ receptor expression, as determined by Western blot, in different breast cancer cell lines from human origin. MDA-MB-231 and MCF-7 cells were used as Her2-negative controls. U373-MG and Jurkat cells were used as positive controls for $CB_1$ and $CB_2$ receptor expression, respectively. (C) Viability of BT474 and SkBr3 cells, as determined by the MTT colorimetric test, in response to 6 μM (BT474) or 3 μM THC (SkBr3) with or without 2 μM SR144528 (SR) for 72 h. Data are expressed as % of vehicle-treated cells, set at 100%. *, p<0.05; **, p<0.01 vs vehicle-treated cells; #, p<0.05 vs THC-treated cells.

Figure 2: Viability of SkBr3 (left panel) and BT474 cells (right panel), as determined by the MTT colorimetric test, in response to increasing concentrations of THC or CBD for 72 h. Data are expressed as % of vehicle-treated cells, set at 100%. n=3.

Figure 3: Viability of BT474 cells, as determined by the MTT colorimetric test, in response to the indicated concentrations of THC and CBD, alone or in combination, for 72 h. Data are expressed as % of vehicle-treated cells, set at 100%. n=3.

Figure 4: Isobologram for 50%, 75% and 90% cell death in BT474 cells, as calculated with CalcuSyn v2.0 software. n=3.

Figure 5: Subcutaneous xenografts were generated from BT474 cells and animals were treated as indicated in the figure. Graph represents mean tumour volume.

Figure 6: Subcutaneous xenografts were generated from BT474 cells and animals were orally treated with the indicated drugs. Graph represents mean tumour volume.

Figure 7: Subcutaneous xenografts were generated from BT474 cells and animals were orally or IP treated with the indicated drugs. Graph represents mean tumour volume.

Figure 8: Viability of BT474 cells, as determined by the MTT colorimetric test, in response to THC or CBD, alone or in combination at the indicated ratios, for 72 h. Data are expressed as % of vehicle-treated cells, set at 100%. n=4. For the combinations, the final cannabinoid concentration is the indicated in the X axis. For example, in the set of bars corresponding to final concentration 1 μM, cells challenged with a 1:10 ratio received 0.09 μM THC and 0.91 μM CBD.

Figure 9: Viability of the indicated cells (see the Methods section for details), as determined by the MTT colorimetric test, in response to increasing concentrations of THC (left panel) or CBD (right panel) for 72 h. Data are expressed as % of vehicle-treated cells, set at 100%. n=3.

Figure 10: Viability of the indicated cells as determined by the MTT colorimetric test, in response to increasing concentrations of THC (left panel) or CBD (right panel) for 72 h. Data are expressed as % of vehicle-treated cells, set at 100%. n=3.

Figure 11: Viability of MDA-MB-231 cells, as determined by the MTT colorimetric test, in response to the indicated concentrations of THC and CBD, alone or in combination, for 48 h. Data are expressed as % of vehicle-treated cells, set at 100%. n>3.

Figure 12: Isobologram for effect doses 50 (ED50), 75 (ED75) and 90 (ED90) in MDA-MB-231 cells, as calculated with CalcuSyn v2.0 software. Effect dose X is defined as the cannabinoid concentration that induces X% cell death. Combination index (CI) values obtained for the corresponding EDs are shown. n>3.

Figure 13: Viability of MDA-MB-231 cells stably overexpressing the oncogene Her2, as determined by the MTT colorimetric test, in response to the indicated concentrations of THC and CBD, alone or in combination, for 48 h. Data are expressed as % of vehicle-treated cells, set at 100%. n>3.

Figure 14: Isobologram for effect doses 50 (ED50), 75 (ED75) and 90 (ED90) in MDA-MB-231 cells stably overexpressing Her2, as calculated with CalcuSyn v2.0 software. Effect dose X is defined as the cannabinoid concentration that induces X% cell death. Combination index (CI) values obtained for the corresponding EDs are shown. n>3.

Figure 15: Viability of a trastuzumab resistant clone of MDA-MB-231 cells stably overexpressing Her2, as determined by the MTT colorimetric test, in response to the indicated concentrations of THC and CBD, alone or in combination, for 48 h. Data are expressed as % of vehicle-treated cells, set at 100%. n>3.

Figure 16: Isobologram for effect doses 50 (ED50), 75 (ED75) and 90 (ED90) in a clone of trastuzumab resistant MDA-MB-231 cells stably overexpressing Her2, as calculated with CalcuSyn v2.0 software. Effect dose X is defined as the cannabinoid concentration that induces X% cell death. Combination index (CI) values obtained for the corresponding EDs are shown. n>3.

Figure 17: Viability of a highly metastatic clone of MDA-MB-231 cells stably overexpressing Her2, as determined by the MTT colorimetric test, in response to the indicated concentrations of THC and CBD, alone or in combination, for 48 h. Data are expressed as % of vehicle-treated cells, set at 100%. n>3.

Figure 18: Isobologram for effect doses 50 (ED50), 75 (ED75) and 90 (ED90) in a clone of highly metastatic MDA-MB-231 cells stably overexpressing Her2, as calculated with CalcuSyn v2.0 software. Effect dose X is defined as the cannabinoid concentration that induces X% cell death. Combination index (CI) values obtained for the corresponding EDs are shown. n>3.

Figure 19: Subcutaneous xenografts were generated from BT474 cells and animals were treated as indicated in the legend (see the Methods section for experimental details). Both THC and CBD were administered in their BDS forms. Graph represents mean tumour volume.

## DETAILED DESCRIPTION

The Examples below demonstrate the effectiveness of the cannabinoids THC and CBD in Her2-positive breast cancer. This type of breast cancer represents a subset of breast tumours characterized by very aggressive clinical courses. These tumours are currently treated with trastuzumab, a humanized monoclonal antibody against Her2.

The examples which concern THC for the treatment of Her-positive breast cancer are reference examples, and not part of the invention.

## EXAMPLE 1: *IN VITRO* EFFECTS OF TETRAHYDROCANNABINOL (THC) AND CANNABIDIOL (CBD) ON HER2-POSITIVE BREAST CANCER CELL LINES

### Materials and Methods

The effect of THC and CBD, alone and in combination, on the viability of two different Her2-positive human breast cancer cell lines: SkBr3 and BT474 were tested. The cannabinoid THC lies outside the scope of the claimed invention.

Briefly, cells were incubated in RPMI (SkBr3) or DMEM (BT474) supplemented with 10% foetal bovine serum. After 12 hours of serum starvation, the cells were challenged with different concentrations of THC or CBD (or the corresponding vehicle, DMSO) for 72 h. Cell viability was then determined by the colorimetric MTT test.

The analysis of the combined drug effect was performed with the CalcuSyn v2.0 software. To determine whether the combination of THC and CBD was additive or synergistic, we calculated different combination indexes (CIs) using the algorithm by Chou and Talalay with the CalcuSyn v2.0 software.

### Results

The cell lines BT474 and SkBr3 were firstly demonstrated to express Her2 (Figure 1A) and $CB_2$ receptors (Figure 1B), and that the cell lines viability decreases upon exposure to THC (Figure 1C) and that this effect is mediated by the activation of $CB_2$ receptors since it is prevented by the $CB_2$ selective antagonist SR144528 (Figure 1C).

The two cell lines were then challenged with different concentrations of THC and CBD. As shown in Figure 2, SkBr3 and BT474 cells reduced their viability in response to THC and CBD in a concentration-related manner. In quantitative terms, the effect was virtually identical for both cannabinoids.

The BT474 cells were then exposed to different concentrations of a combination of THC and CBD in a 1:1 ratio. Interestingly, we observed that the combination of low concentrations (< 1 μM) of the cannabinoids (concentrations with no effect on cell viability when administered alone) significantly diminished the viability of the cell cultures (Figure 3).

We obtained the CI values shown in Table 1.1 below, all of the CI values were less than 1, indicating that these compounds were producing a strong synergistic effect.

**Table 1.1 CI Values of the combination of THC and CBD in breast cancer cell viability**

| | ED50 | ED75 | ED90 |
|---|---|---|---|
| **CI** | 0.52 | 0.55 | 0.57 |

This synergism can be also observed in the isobologram shown in Figure 4. This graph was generated by selecting the cannabinoid concentrations that decrease cell viability by 50% (ED50), 75% (ED75) and 90% (ED90). For each ED, the software (i) plots a line connecting the concentrations of THC and CBD that produce that particular effect when administered as single agents, and (ii) generates a point that indicates the concentration of THC and CBD, applied as a combination, required to produce such effect. If the line is straight, this means that the two compounds are producing a simple additive effect and the point should appear within it. In the example demonstrated herein, the lines are curved downwards and the points appear below them, indicating that lower amounts of cannabinoids are required to get the same effects if they are administered as a combination. Both observations confirm that the combination of THC and CBD has synergic effects.

**Conclusion**

The results demonstrate that a low dose of a combination of THC and CBD have significant antiproliferative effects in human Her2-positive breast cancer cells.
Importantly a synergic effect was achieved with half the dose of THC that was required when administered alone. This observation may have important implications for the development of cannabinoid-based therapies for breast tumours, as the dose of THC could be reduced, in consequence diminishing or removing any potential side effects, without affecting its potency.
These data provide strong evidence for the use of cannabinoid-based therapies for the management of Her2-positive breast cancer, which represents a subset of breast tumours characterized by very aggressive clinical courses, reduced responsiveness to conventional and targeted therapies and high relapsing frequencies.

**EXAMPLE 2: *IN VITRO* EFFECTS OF TETRAHYDROCANNABINOL (THC) AND CANNABIDIOL (CBD) ON AN HER2-POSITIVE BREAST CANCER CELL LINE**

**Materials and Methods**

Ectopic xenografts were generated by subcutaneous injection of $5x10^6$ human BT474 cells (Her2-positive human breast cancer cells). Animals were divided into 11 experimental groups (8 animals/group) and when tumours reached 200 $mm^3$, they were treated as detailed in Table 2.1 below for a period of 4 weeks.

**Table 2.1 Treatment groups**

| GROUP | DRUG | ROUTE | VOLUME | PATTERN |
|---|---|---|---|---|
| VEHICLE | Sesame oil | Oral | 300 μL | 3 days/week |
| | PBS | IP | 50 μL | 2 days/week |
| THC-BDS | 45mg/Kg THC-BDS | Oral | 300 μL | 3 days/week |
| | PBS | IP | 50 μL | 2 days/week |
| CBD-BDS | 45mg/Kg CBD-BDS | Oral | 300 μL | 3 days/week |
| | PBS | IP | 50 μL | 2 days/week |
| THC-BDS + CBD-BDS | 22.5mg/Kg THC-BDS<br>22.5mg/Kq CBD-BDS | Oral | 300 μL | 3 days/week |
| | PBS | IP | 50 μL | 2 days/week |
| THC-BDS + CBD-BDS | 45mg/Kg THC-BDS<br>45mg/Kg CBD-BDS | Oral | 300 μL | 3 days/week |
| | PBS | IP | 50 μL | 2 days/week |
| TRASTUZUMAB | Sesame oil | Oral | 300 μL | 3 days/week |
| | Trastuzumab | IP | 50 μL | 2 days/week |
| TRASTUZUMAB +THC-BDS | 45mg/Kg THC-BDS | Oral | 300 μL | 3 days/week |
| | Trastuzumab | IP | 50 μL | 2 days/week |

(continued)

| GROUP | DRUG | ROUTE | VOLUME | PATTERN |
|---|---|---|---|---|
| TRASTUZUMAB +CBD-BDS | 45mg/Kg CBD-BDS | Oral | 300 μL | 3 days/week |
| | Trastuzumab | IP | 50 μL | 2 days/week |
| TRASTUZUMAB + THC-BDS + CBD-BDS | 22.5mg/Kg THC-BDS<br>22.5mg/Kg CBD-BDS | Oral | 300 μL | 3 days/week |
| | Trastuzumab | IP | 50 μL | 2 days/week |
| VEHICLE | PBS+5% BSA+2.5% DMSO | IP | 100 μL | 3 days/week |
| THC-BDS | 15mg/Kg THC-BDS | IP | 100 μL | 3 days/week |

Different routes of administration were tested along with different combinations of THC, CBD and trastuzumab. The cannabinoid THC lies outside the scope of the claimed invention.

The cannabinoids THC and CBD were in the form of botanical drug substances (BDS) whereby the major cannabinoid is present along with other cannabinoid components and a non-cannabinoid fraction.

The relative amounts of the different cannabinoids in the THC and CBD BDSs are shown in Table 2.2 below. It will be appreciated that variations of plus or minus 25% (w/w) can occur with BDS components depending on the method of extraction used.

**Table 2.2 Cannabinoid and Non-cannabinoid components of THC and CBD BDS**

| | THC-BDS (% w/w) | CBD-BDS (% w/w) |
|---|---|---|
| **Cannabinoid Fraction:** | | |
| **Major Cannabinoid:** | | |
| THC | 63.0 - 78.0 | n/a |
| CBD | n/a | 57.0 - 72.0 |
| **Minor / Other Cannabinoids:** | | |
| THC | n/a | 2.0 - 6.5 |
| CBD | 0.1 - 2.5 | n/a |
| Cannabigerol (CBG) | 1.0 - 2.0 | 0.8 - 6.5 |
| Cannabichromene (CBC) | 0.8 - 2.2 | 3.0 - 6.5 |
| Tetrahydrocannabivarin (THCV) | 0.4 - 1.0 | - |
| Tetrahydrocannabinolic acid (THCA) | <2.0 | - |
| Cannabidivarin | - | 1.0 - 2.0 |
| Cannabidiolic acid | - | <2.0 |
| **Non-cannabinoid fraction:** | | |
| **Terpenes:** | | |
| Monoterpenes | 0.7 | 0.4 |
| Di/tri-terpenes | 0.6 | 0.4 |
| Sesquiterpenes | 1.7 | 2.0 |
| Other terpenes | <3.0 | <3.0 |

(continued)

| Other minor plant derived components including: | | |
|---|---|---|
| Sterols<br>Triglycerides<br>Alkanes<br>Squalene<br>Tocopherol<br>Carotenoids | 6.3 - 26.7 | 1.7 - 28.4 |

Tumours were routinely measured during this period with an external calliper, and their volume was calculated as $(4\pi/3) \times (\text{width}/2)^2 \times (\text{length}/2)$.

## Results

Oral administration of THC significantly reduced tumour growth and interestingly this route of administration is more effective than IP administration (Figure 5).

The results also clearly show an important anti-tumour effect of CBD in this model (Figure 6). In addition the combination of THC and CBD in a 1:1 ratio had a greater effect than each cannabinoid alone (Figure 6).

Both THC and CBD were able to improve the anti-tumour action of trastuzumab, especially when administered in combination (Figure 7). The best anti-tumour response was obtained with the high dose of the combination of THC and CBD without trastuzumab (Figure 7).

## Conclusion

These data demonstrate that human Her2-positive cancer cells are sensitive to phytocannabinoids *in vivo.* Indeed the combination of THC and CBD were more effective than the standard treatment trastuzumab which leads to a positive indication for these cannabinoids in the treatment of aggressive types of breast cancer.

## EXAMPLE 3: *IN VIVO* EFFECTS OF DIFFERENT RATIOS OF TETRAHYDROCANNABINOL (THC) AND CANNABIDIOL (CBD) ON AN HER2-POSITIVE BREAST CANCER CELL LINE

CBD has been shown to have significant anti-tumour properties in breast cancer. Since this compound has a very safe profile, experiments were performed to determine whether the proportion of the psychoactive compound THC can be decreased in the combination treatments

### Materials and Methods

The effect of THC and CBD, alone and in combination at different ratios, on the viability of BT474 cells was examined. The cannabinoid THC lies outside the scope of the claimed invention.

Cells were incubated in DMEM supplemented with 10% foetal bovine serum. After 12 hours of serum starvation, cells were challenged for 72 h as described in Table 3.1. Cell viability was then determined by the colorimetric MTT test.

**Table 3.1 Cannabinoid concentrations**

| Total cannabinoid (μM) | CONDITION | THC (μM) | CBD (μM) |
|---|---|---|---|
| 0.5 | THC | 0.5 | - |
| | CBD | - | 0.5 |
| | THC:CBD (1:1) | 0.25 | 0.25 |
| | THC:CBD (1:5) | 0.08 | 0.42 |
| | THC:CBD (1:7) | 0.06 | 0.44 |
| | THC:CBD (1:10) | 0.05 | 0.45 |

(continued)

| Total cannabinoid (μM) | CONDITION | THC (μM) | CBD (μM) |
|---|---|---|---|
| 1.0 | THC | 1.0 | - |
| | CBD | - | 1.0 |
| | THC:CBD (1:1) | 0.5 | 0.5 |
| | THC:CBD (1:5) | 0.16 | 0.84 |
| | THC:CBD (1:7) | 0.12 | 0.88 |
| | THC:CBD (1:10) | 0.1 | 0.9 |
| 1.5 | THC | 1.5 | - |
| | CBD | - | 1.5 |
| | THC:CBD (1:1) | 0.75 | 0.75 |
| | THC:CBD (1:5) | 0.24 | 1.26 |
| | THC:CBD (1:7) | 0.18 | 1.32 |
| | THC:CBD (1:10) | 0.15 | 1.35 |

**Results**

These data indicate that combinations of THC and CBD in which THC proportion was as low as 1:10 were as effective as the 1:1 ratios in decreasing cell viability (Figure 8).

**Conclusions**

Human Her2-positive cancer cells are sensitive to phytocannabinoids both in cell cultures and *in vivo.* It is noteworthy that decreasing the ratio of THC to as low as 1:10 (THC:CBD) did not make any difference in the effectiveness of the combination of the cannabinoids.

As such ratioed combinations of THC and CBD could be used to treat aggressive forms of breast cancer with very few, if any side effects.

**EXAMPLE 4: EFFECTS OF TETRAHYDROCANNABINOL (THC) AND CANNABIDIOL (CBD) ON A TRASTUZUMAB-RESISTANT BREAST CANCER CELL LINE**

**Materials and Methods**

The effect of THC and CBD on the viability of a series of Her2-positive human breast cancer cell lines: MDA-MB-231 cells that ectopically overexpress Her2 (231-Her2), a highly metastatic version of 231-Her2 (231-Met), and three different trastuzumab-resistant 231-Her2-derived cell lines (TrR1, TrR2 and TrR3). The cannabinoid THC lies outside the scope of the claimed invention.

Cells were incubated in DMEM supplemented with 10% foetal bovine serum. After 12 hours of serum starvation, cells were challenged with different concentrations of THC or CBD (or the corresponding vehicle, DMSO) for 72 h. Cell viability was then determined by the colorimetric MTT test.

**Results**

As shown in Figure 9, the highly metastatic 231-Her2 cell line decreased its viability in response to increasing concentrations of THC or CBD, with $IC_{50}$ values virtually identical for both compounds.

Similar studies were performed with trastuzumab-resistant cells and the results were analogous: these cell lines decreased their viability in response to phytocannabinoids in a concentration dependent manner (Figure 10).

**Conclusion**

Together, these results suggest that, whatever molecular changes these cells have suffered to become extremely aggressive (highly metastatic or resistant to trastuzumab), phytocannabinoids are still effect agents in decreasing these cells viability and ability to metastasize.

**EXAMPLE 5: EFFECTS OF A COMBINATION OF TETRAHYDROCANNABINOL (THC) AND CANNABIDIOL (CBD) ON A TRASTUZUMAB-RESISTANT BREAST CANCER CELL LINE**

**Materials and Methods**

The previous Examples demonstrate that 1:9 THC:CBD is as effective as the 1:1 ratio in reducing BT474 cell viability *in vitro.* This data demonstrating the effect of 1:9 THC:CBD combinations in BT474 cells *in vivo* is provided in this example. In addition the effect of such a combination on trastuzumab-resistant and in highly metastatic Her2-overexpressing MDA-MB-231 cells *in vitro* is also demonstrated. The cannabinoid THC lies outside the scope of the claimed invention.

The effect of a 1:9 combination of THC:CBD on Her2-positive cancer cells *in vitro,* was demonstrated by challenging the following cell lines with the cannabinoid concentrations indicated in the corresponding figure legends:

- F MDA-MB-231 Triple negative (no Her2 overexpression) human breast cancer cell line
- 231-Her2 MDA-MB-231 cells stably overexpressing Her2
- 231-Her2-Met Highly metastatic 231-Her2-derived cell line
- 231-Her2-TrR Trastuzumab resistant 231-Her2-derived cell line

Cells were incubated in the presence of cannabinoids for 48h and cell viability was assessed by the MTT colorimetric test.

To study the effect of different THC:CBD ratios on human Her2-positive breast cancer *in vivo,* we generated ectopic xenografts in immune-deficient female mice by subcutaneous injection of $5x10^6$ BT474 cells. Animals were divided into 7 experimental groups (8 animals/group) and when tumours reached 200 $mm^3$ (typically 40 days after cancer cell injection), they started to be treated as indicated in the table below.

| GROUP | DRUG | ROUTE OF ADMINISTRATION | VOLUME | PATTERN OF ADMINISTRATION |
|---|---|---|---|---|
| VEHICLE | Sesame oil | Oral | 300 μL | 3 days/week |
| THC 45 | 45 mg/Kg THC-BDS | | | |
| CBD 45 | 45 mg/Kg THC-BDS | | | |
| THC:CBD (1: 1) | 90 mg/Kg total cannabinoid (45 mg/Kg THC-BDS + 45 mg/Kg THC-BDS) | | | |
| THC 9 | 9 mg/Kg THC-BDS | | | |
| CBD 81 | 81 mg/Kg CBD-BDS | | | |
| THC:CBD (1: 9) | 90 mg/Kg total cannabinoid (9 mg/Kg THC-BDS + 81 mg/Kg THC-BDS) | | | |

Tumours were routinely measured with external calliper, and volume was calculated as $(4\pi/3) \times (\text{width}/2)^2 \times (\text{length}/2)$.

**Results**

Previous examples demonstrated that THC:CBD combinations in which THC represents just one tenth of the total cannabinoid concentration were as effective as 1:1 ratios in reducing the viability of human breast cancer cells naturally overexpressing the oncogene Her2 (BT474 cells).

This example demonstrates similar experiments with MDA-MB-231-derived cell lines. MDA-MB-231 cells are widely used in breast cancer studies due to their ability to form tumours and metastasis *in vivo.* However, this cell line does not express Her2. To convert MDA-MB-231 cells into a Her2-positive breast cancer model, we used 231 cells stably transfected with Her2 (231-Her2) and two clones derived from them: one showing resistance to trastuzumab treatment (231-Her2-TrR) and another with an increased metastatic potential (231-Her2-Met).

Figure 11 demonstrates that triple-negative MDA-MB-231 cells are sensitive to both THC and CBD. The combination of the two phytocannabinoids in a 1:1 proportion has additive effects, as suggested from Figure 11 and demonstrated in Figure 12. Similar effects were observed when the THC proportion was reduced to a 1:9 ratio (Figures 11 and 12).

Overexpression of the Her2 oncogene in MDA-MB-231 cells did not alter responsiveness to phytocannabinoids (Figures 13 and 14). Thus, the viability of these cells decreases in response to increasing concentrations of THC and CBD alone and both 1:1 and 1:9 combinations had the same additive effects (Figures 13 and 14).

Trastuzumab resistant (Figures 15 and 16) and highly metastatic (Figures 17 and 18) Her2-positive cells behave as their controls (231-Her2) in terms of cell viability in response to phytocannabinoids alone or in combination. As for the 231 and 231-Her2 cell lines, the 1:9 THC:CBD ratios were as effective as the 1:1 combinations (Figures 15-18).

The effect of phytocannabinoid combinations on Her2-positive cells *in vivo* was also examined. BT474 subcutaneous xenografts decreased their growth in response to THC-BDS and CBD-BDS alone (Figure 19). The combination of the two cannabinoids in a 1:1 ratio produces the same effect than the compounds alone. Interestingly, the 1:9 combination induced the strongest anti-tumour response (Figure 19).

## Conclusion

These data demonstrate that MDA-MB-231 and 231-Her2 cells are equally sensitive to THC and CBD alone; that 1:1 THC:CBD combinations have additive effects on 231 and 231-Her2 cells *in vitro*; and that 1:9 THC:CBD combinations are as effective as 1:1 combinations in decreasing cell viability.

Despite the molecular changes suffered to become highly aggressive, trastuzumab-resistant and highly metastatic Her2-positive cells retain their capacity to respond to phytocannabinoids as described in the example above.

It is also demonstrated that a 1:9 THC:CBD combination is more effective than 1:1 combinations in decreasing BT474 xenograft growth.

## References

Caffarel, M.M., Andradas, C., Mira, E., Perez-Gomez, E., Cerutti, C., Moreno-Bueno, G., Flores, J.M., Garcia-Real, I., Palacios, J., Manes, S., et al. 2010. Cannabinoids reduce ErbB2-driven breast cancer progression through Akt inhibition. Mol Cancer 9:196.

Ligresti, A., Moriello, A.S., Starowicz, K., Matias, I., Pisanti, S., De Petrocellis, L., Laezza, C., Portella, G., Bifulco, M., and Di Marzo, V. 2006. Antitumor activity of plant cannabinoids with emphasis on the effect of cannabidiol on human breast carcinoma. J Pharmacol Exp Ther 318:1375-1387.

McAllister, S.D., Christian, R.T., Horowitz, M.P., Garcia, A., and Desprez, P.Y. 2007. Cannabidiol as a novel inhibitor of Id-1 gene expression in aggressive breast cancer cells. Mol Cancer Ther 6:2921-2927.

McAllister, S.D., Murase, R., Christian, R.T., Lau, D., Zielinski, A.J., Allison, J., Almanza, C., Pakdel, A., Lee, J., Limbad, C., et al. 2010. Pathways mediating the effects of cannabidiol on the reduction of breast cancer cell proliferation, invasion, and metastasis. Breast Cancer Res Treat.

## Claims

1. Cannabidiol (CBD) for use in the treatment of aggressive breast cancer **characterised by** overexpression of the Her2 gene.

2. CBD for use as claimed in claim 1, wherein the CBD is in the form of a botanical drug substance (BDS).

3. CBD for use as claimed in any of the preceding claims, wherein the CBD is present in an approximate amount of between 1mg and 2000mg.

4. CBD for use as claimed in any of the preceding claims, which further comprises a non-cannabinoid chemotherapeutic agent.

**5.** CBD for use as claimed in any of the preceding claims, wherein the non-cannabinoid chemotherapeutic agent is a monoclonal antibody.

**6.** CBD for use as claimed in claim 5, wherein the non-cannabinoid chemotherapeutic agent is trastuzumab.

## Patentansprüche

**1.** Cannabidiol (CBD) zur Verwendung in der Behandlung von aggressivem Brustkrebs, **gekennzeichnet durch** eine Überexpression des Gens Her2.

**2.** CBD zur Verwendung nach Anspruch 1, wobei die CBD in Form einer botanischen Arzneimittelsubstanz (botanical drug substance, BDS) ist.

**3.** CBD zur Verwendung nach einem der vorherigen Ansprüche, wobei das CBD in einer ungefähren Menge von zwischen 1 mg und 2000 mg vorhanden ist.

**4.** CBD zur Verwendung nach einem der vorherigen Ansprüche, das ferner ein nichtcannabinoides Chemotherapeutikum umfasst.

**5.** CBD zur Verwendung nach einem der vorherigen Ansprüche, wobei das nicht-cannabinoide Chemotherapeutikum ein monoklonaler Antikörper ist.

**6.** CBD zur Verwendung nach Anspruch 5, wobei das nicht-cannabinoide Chemotherapeutikum Trastuzumab ist.

## Revendications

**1.** Cannabidiol (CBD) destiné à être utilisé dans le traitement d'un cancer du sein agressif **caractérisé par** la surexpression du gène Her2.

**2.** CBD destiné à être utilisé selon la revendication 1, dans lequel le CBD est sous la forme d'une substance médicamenteuse botanique (SMB).

**3.** CBD destiné à être utilisé selon l'une quelconque des revendications, dans lequel le CBD est présent dans une quantité approximative située entre 1 mg et 2 000 mg.

**4.** CBD destiné à être utilisé selon l'une quelconque des revendications, qui comprend en outre un agent chimiothérapeutique non cannabinoïde.

**5.** CBD destiné à être utilisé selon l'une quelconque des revendications, dans lequel l'agent chimiothérapeutique non cannabinoïde est un anticorps monoclonal.

**6.** CBD destiné à être utilisé selon la revendication 5, dans lequel l'agent chimiothérapeutique non cannabinoïde est le trastuzumab.

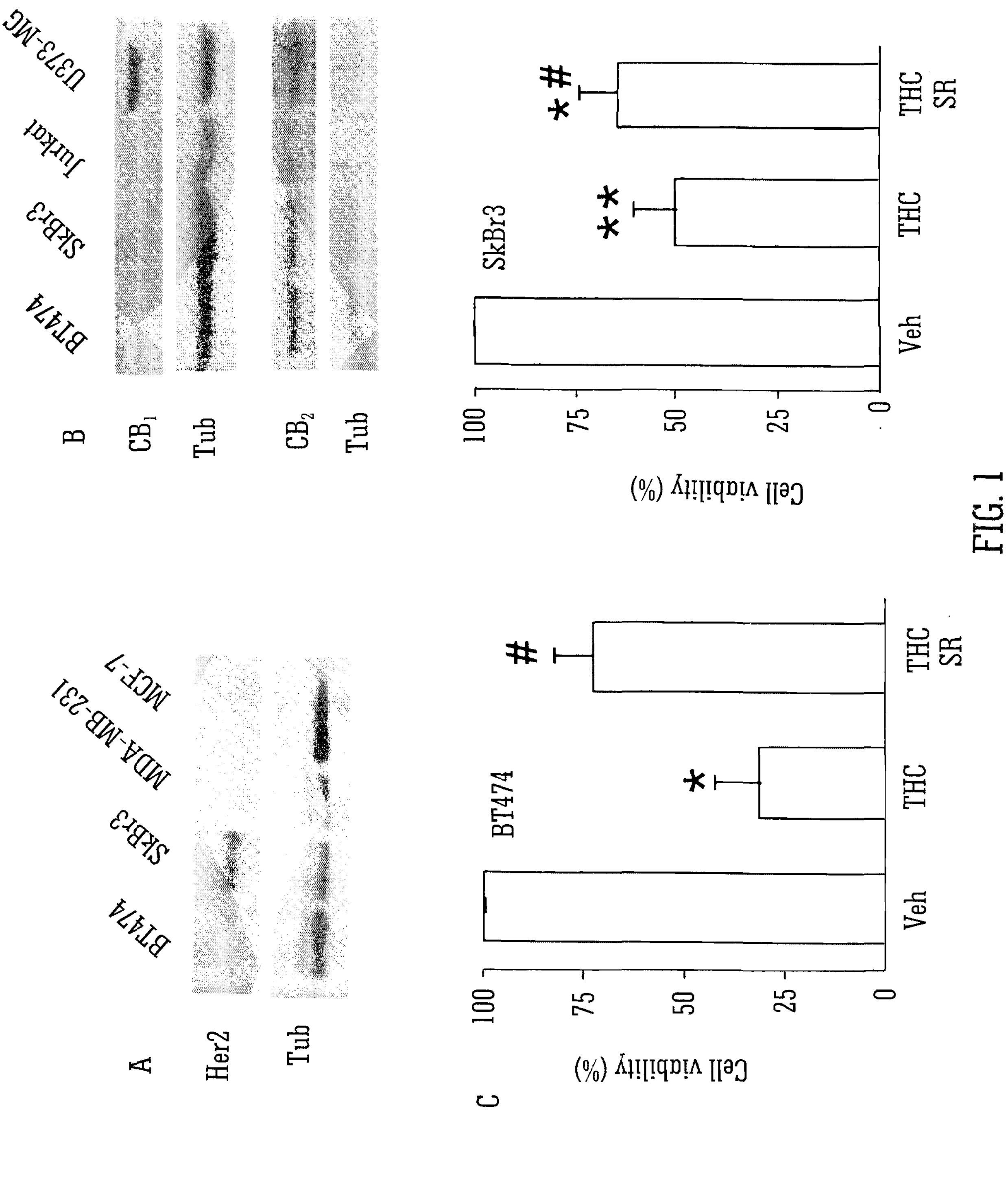
A
BT474
SkBr3
MDA-MB-231
MCF-7
Her2
Tub
B
BT474
SkBr3
Jurkat
U373-MG
CB1
Tub
CB2
Tub
C
Cell viability (%)
100
75
50
25
0
BT474
*
#
Veh
THC
THC SR
SkBr3
**
*#
Veh
THC
THC SR

FIG. 1

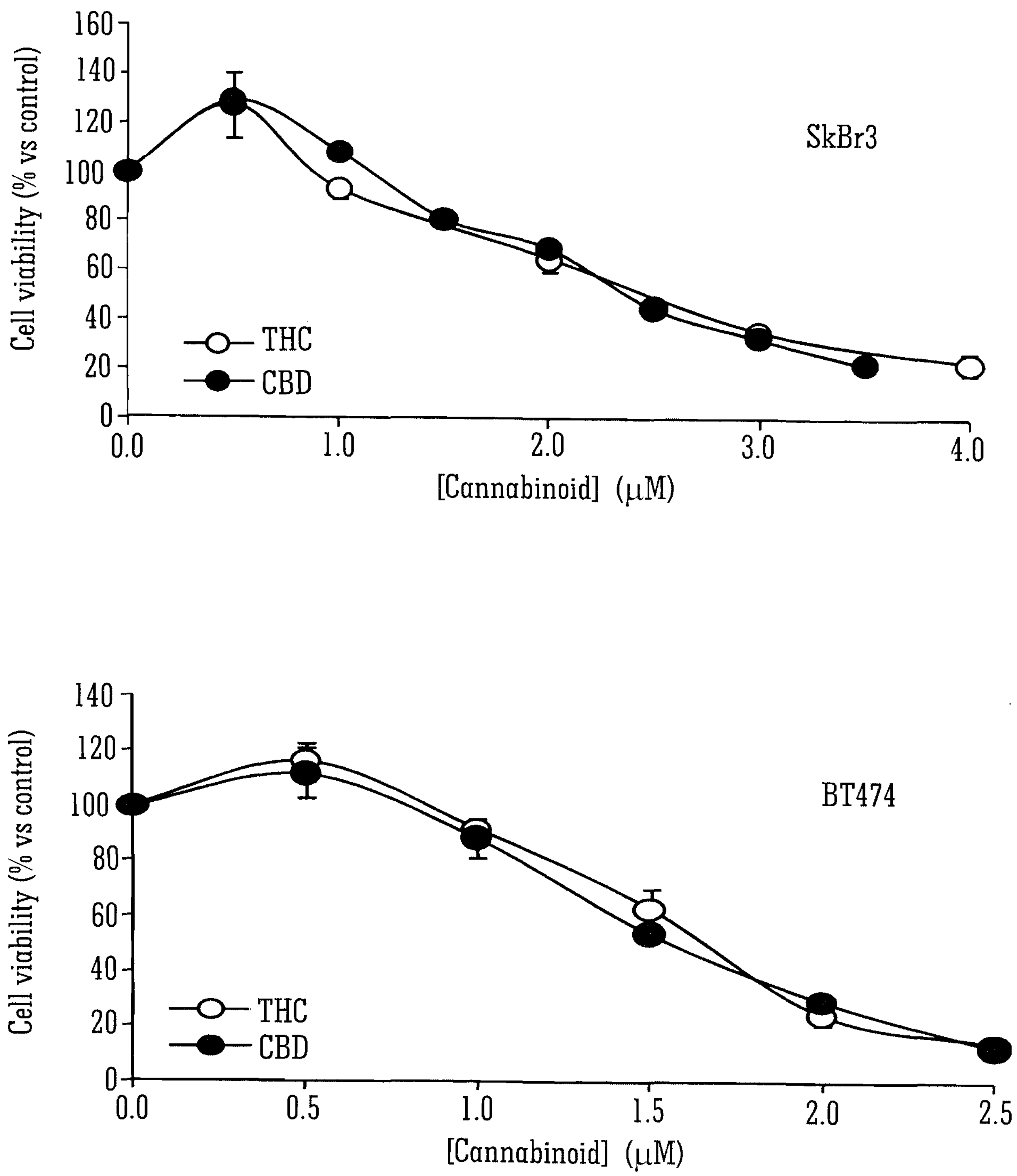
Cell viability (% vs control)
160
140
120
100
80
60
40
20
0
SkBr3
THC
CBD
0.0
1.0
2.0
3.0
4.0
[Cannabinoid] (μM)
Cell viability (% vs control)
140
120
100
80
60
40
20
0
BT474
THC
CBD
0.0
0.5
1.0
1.5
2.0
2.5
[Cannabinoid] (μM)

FIG. 2

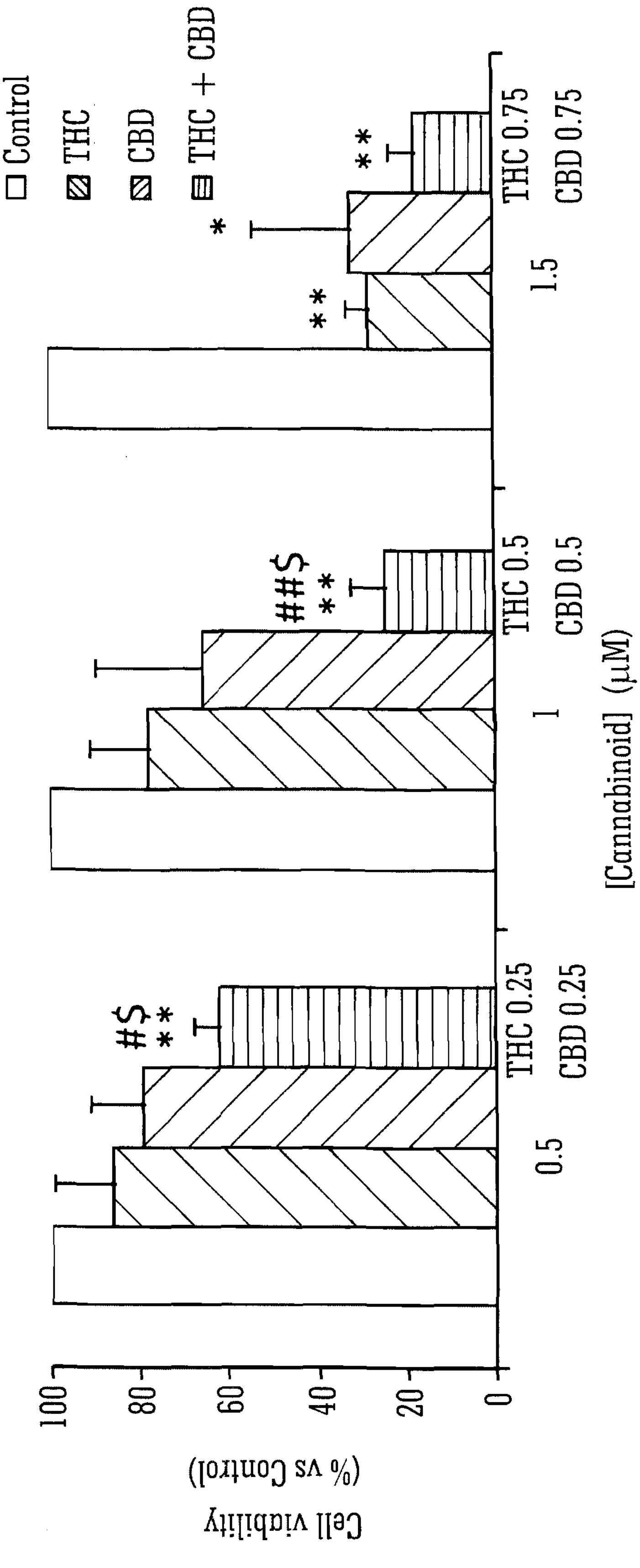
Control
THC
CBD
THC + CBD
Cell viability
(% vs Control)
100
80
60
40
20
0
0.5
THC 0.25
CBD 0.25
1
THC 0.5
CBD 0.5
1.5
THC 0.75
CBD 0.75
[Cannabinoid] (μM)
#$
**
##$
**
**
*
**

FIG. 3

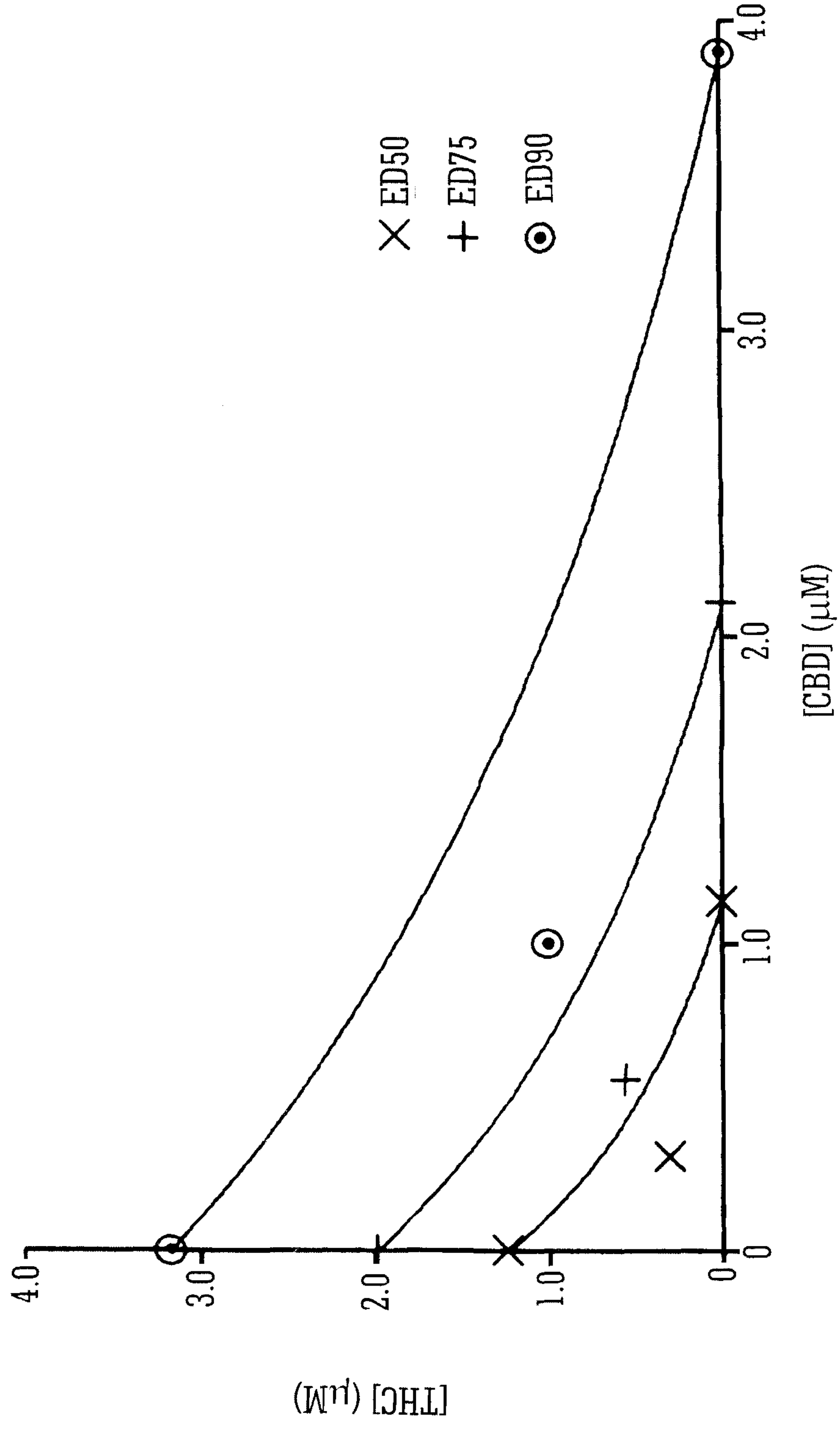


FIG. 4

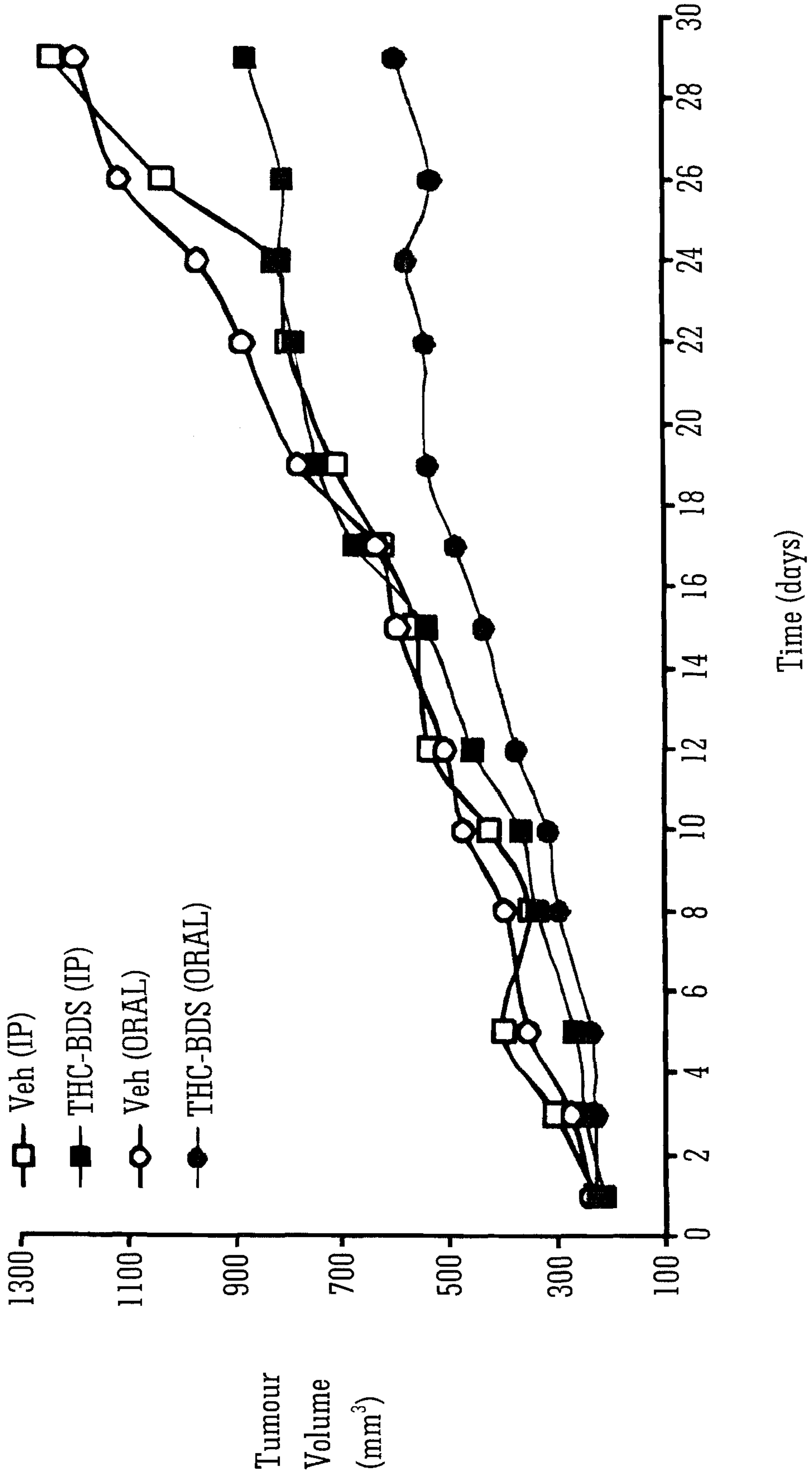
Veh (IP)
THC-BDS (IP)
Veh (ORAL)
THC-BDS (ORAL)
Tumour Volume (mm$^3$)
1300
1100
900
700
500
300
100
0
2
4
6
8
10
12
14
16
18
20
22
24
26
28
30
Time (days)

FIG. 5

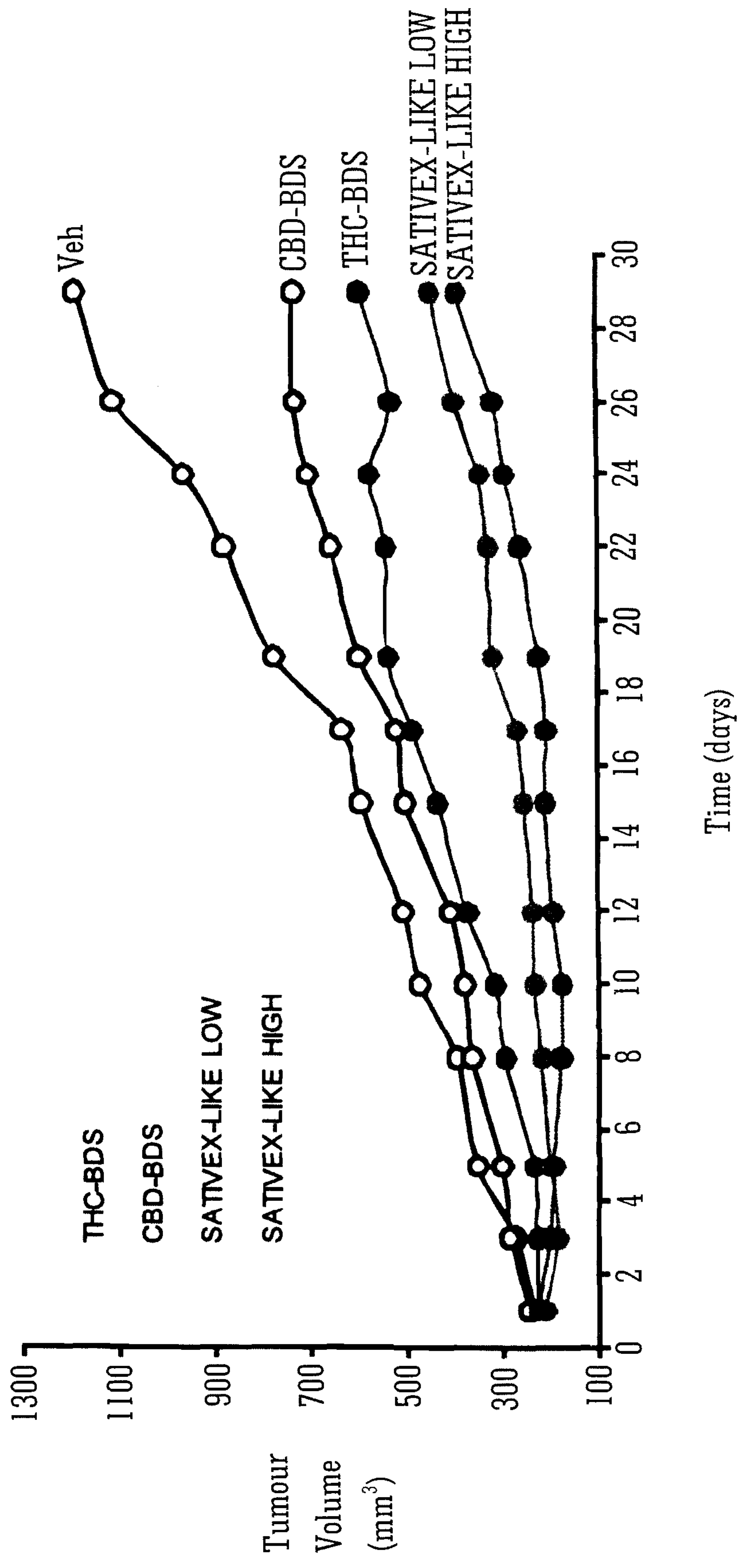

Tumour
Volume
(mm³)
1300
1100
900
700
500
300
100
0
2
4
6
8
10
12
14
16
18
20
22
24
26
28
30
Time (days)
THC-BDS
CBD-BDS
SATIVEX-LIKE LOW
SATIVEX-LIKE HIGH
Veh
CBD-BDS
THC-BDS
SATIVEX-LIKE LOW
SATIVEX-LIKE HIGH


FIG. 6

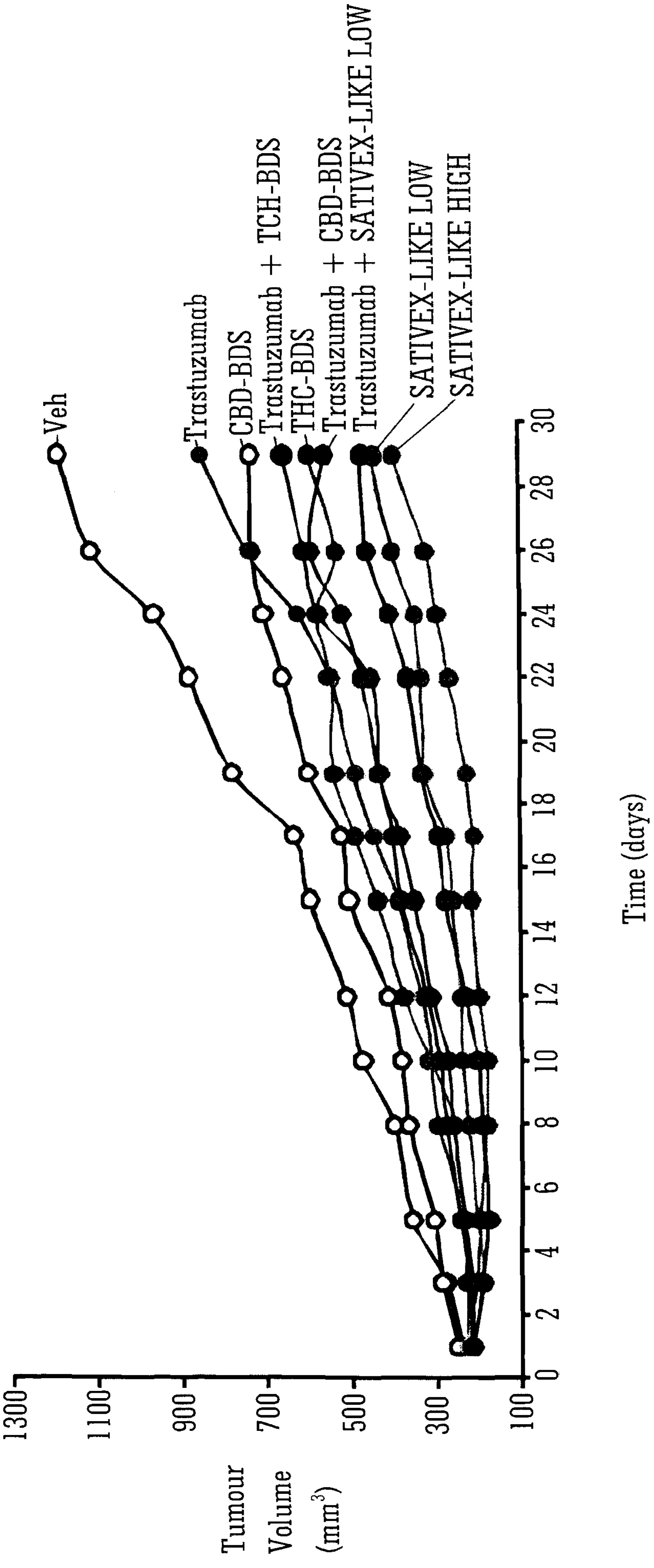

Veh
Trastuzumab
CBD-BDS
Trastuzumab + TCH-BDS
THC-BDS
Trastuzumab + CBD-BDS
Trastuzumab + SATIVEX-LIKE LOW
SATIVEX-LIKE LOW
SATIVEX-LIKE HIGH
Tumour Volume (mm3)
1300
1100
900
700
500
300
100
0
2
4
6
8
10
12
14
16
18
20
22
24
26
28
30
Time (days)


FIG. 7

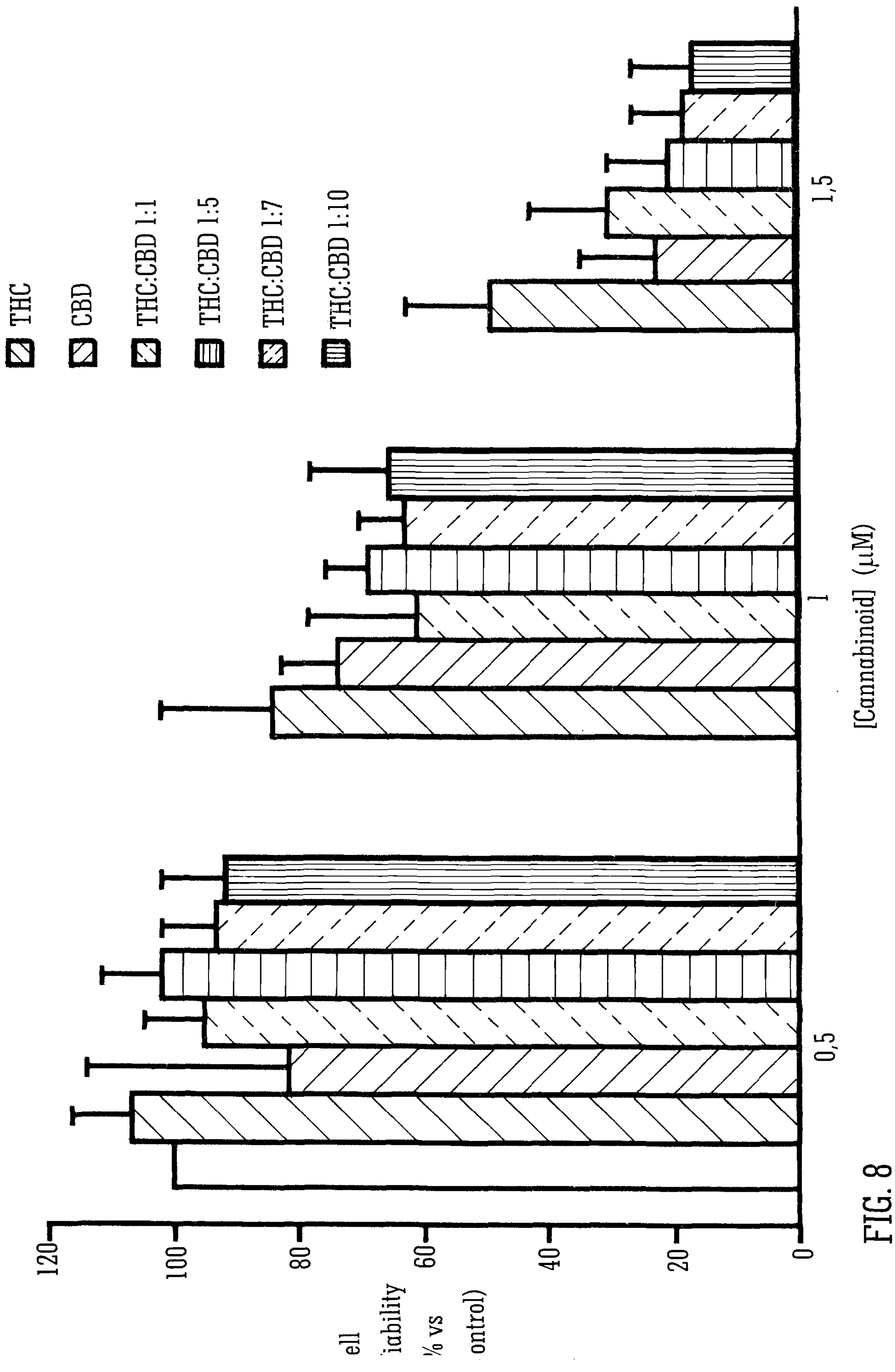
THC
CBD
THC:CBD 1:1
THC:CBD 1:5
THC:CBD 1:7
THC:CBD 1:10
0
20
40
60
80
100
120
ell
iability
% vs
ontrol)
0,5
1
1,5
[Cannabinoid] (μM)

FIG. 8

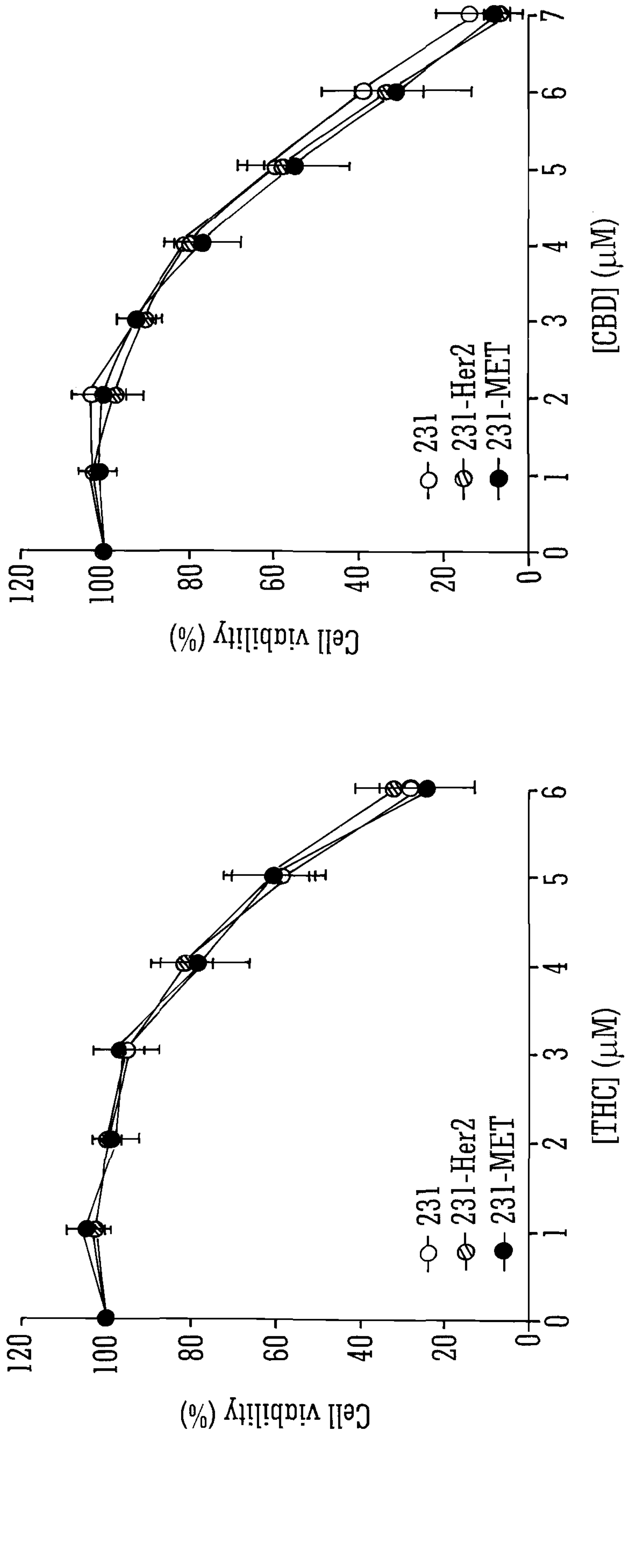
Cell viability (%)
[CBD] (μM)
231
231-Her2
231-MET
Cell viability (%)
[THC] (μM)
231
231-Her2
231-MET

FIG. 9

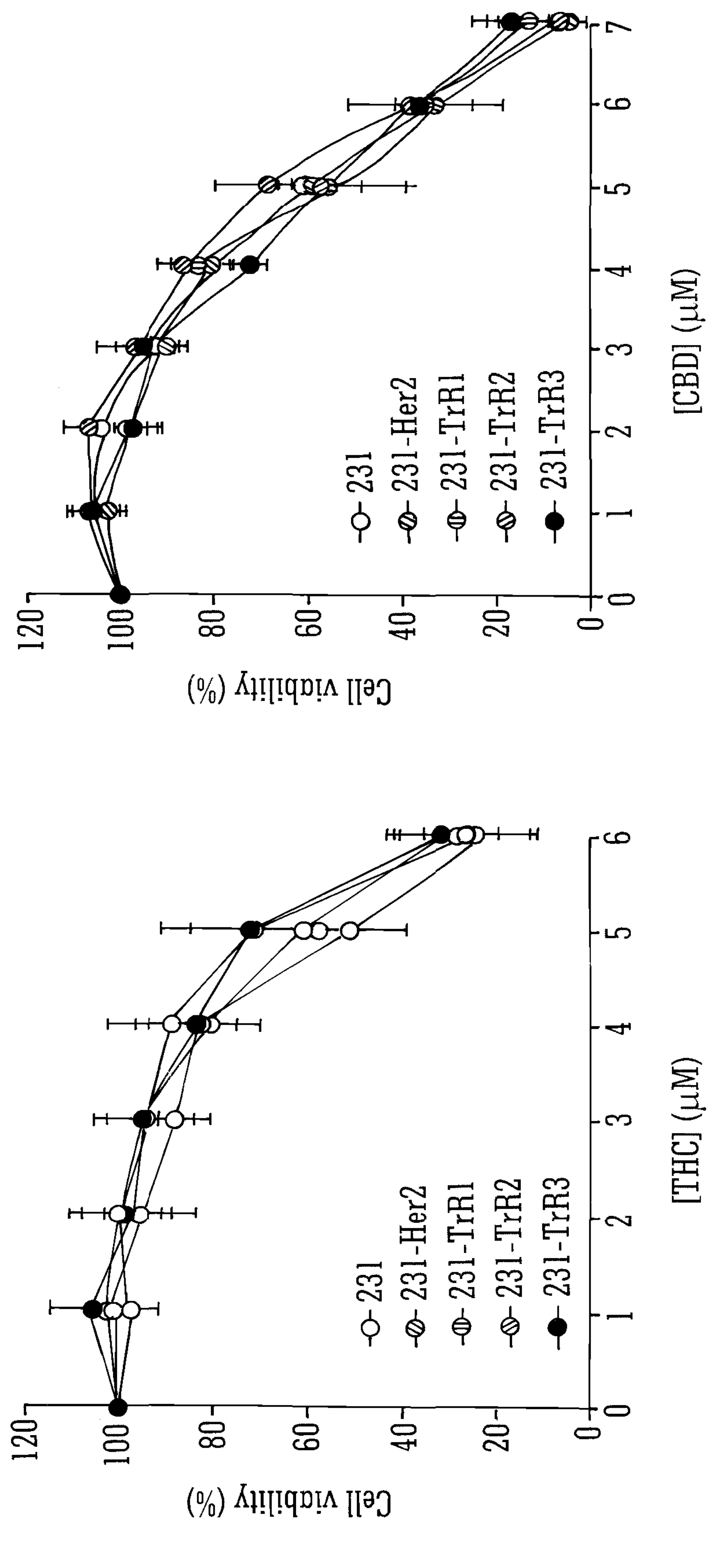
Cell viability (%)
120
100
80
60
40
20
0
0
1
2
3
4
5
6
[THC] (μM)
231
231-Her2
231-TrR1
231-TrR2
231-TrR3
Cell viability (%)
7
[CBD] (μM)

FIG. 10

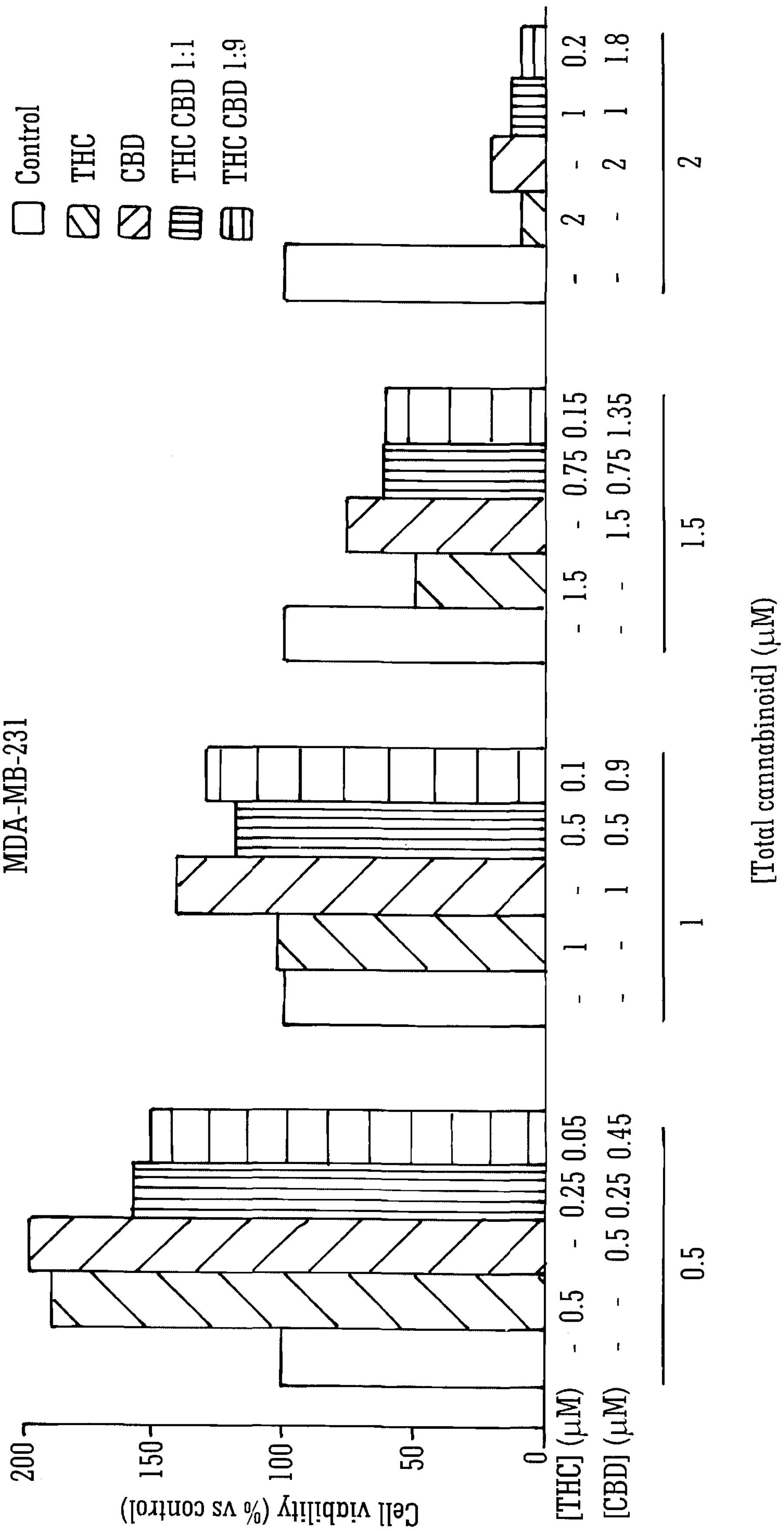

MDA-MB-231
Control
THC
CBD
THC CBD 1:1
THC CBD 1:9
Cell viability (% vs control)
200
150
100
50
0
[THC] (μM) - 0.5 - 0.25 0.05 - 1 - 0.5 0.1 - 1.5 - 0.75 0.15 - 2 - 1 0.2
[CBD] (μM) - - 0.5 0.25 0.45 - - 1 0.5 0.9 - - 1.5 0.75 1.35 - - 2 1 1.8
0.5
1
1.5
2
[Total cannabinoid] (μM)


FIG. 11A

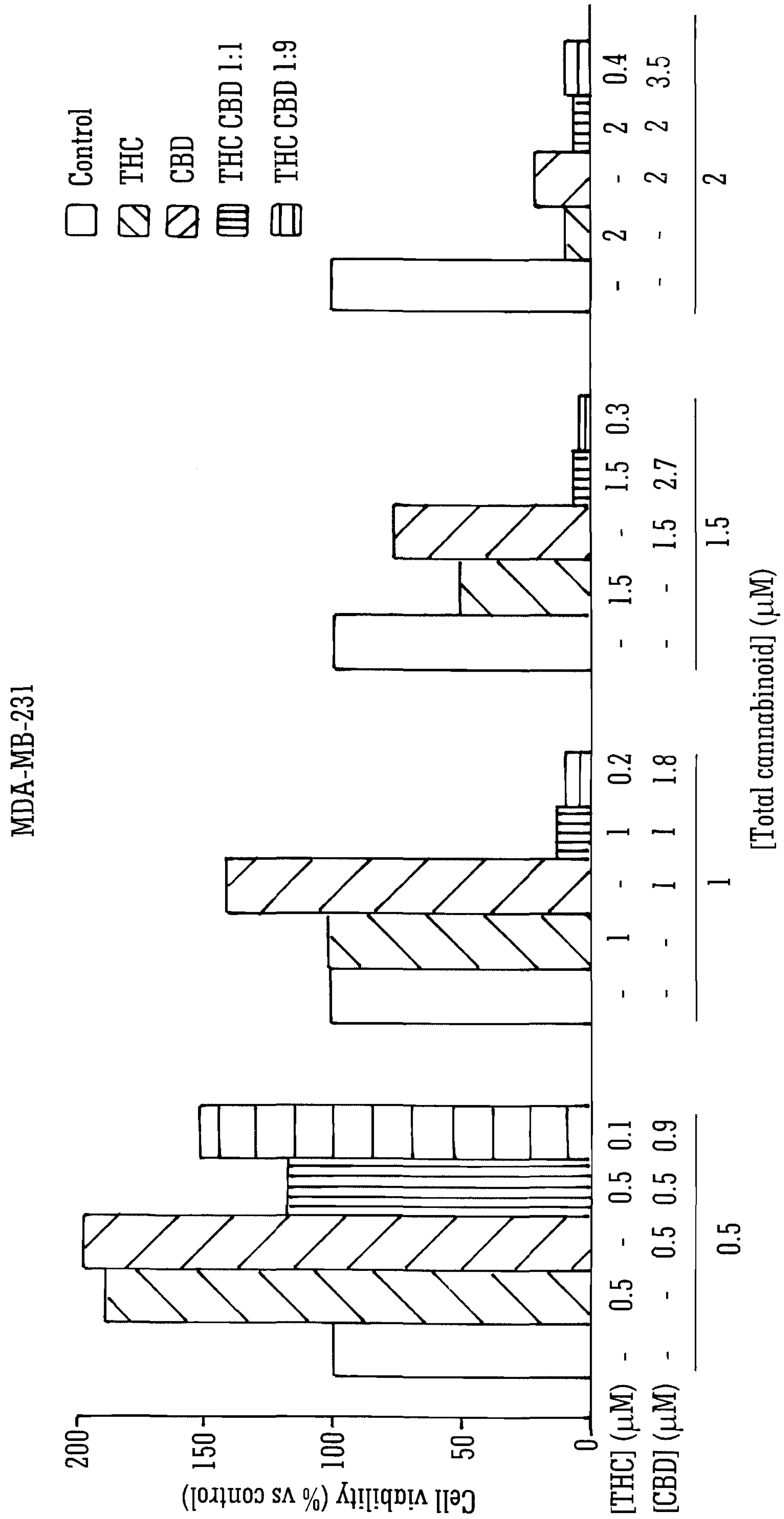
MDA-MB-231
Control
THC
CBD
THC CBD 1:1
THC CBD 1:9
Cell viability (% vs control)
200
150
100
50
0
[THC] (μM) - 0.5 - 0.5 0.1 - 1 - 1 0.2 - 1.5 - 1.5 0.3 - 2 - 2 0.4
[CBD] (μM) - - 0.5 0.5 0.9 - - 1 1 1.8 - - 1.5 2.7 - - 2 2 3.5
0.5
1
1.5
2
[Total cannabinoid] (μM)

FIG.11B

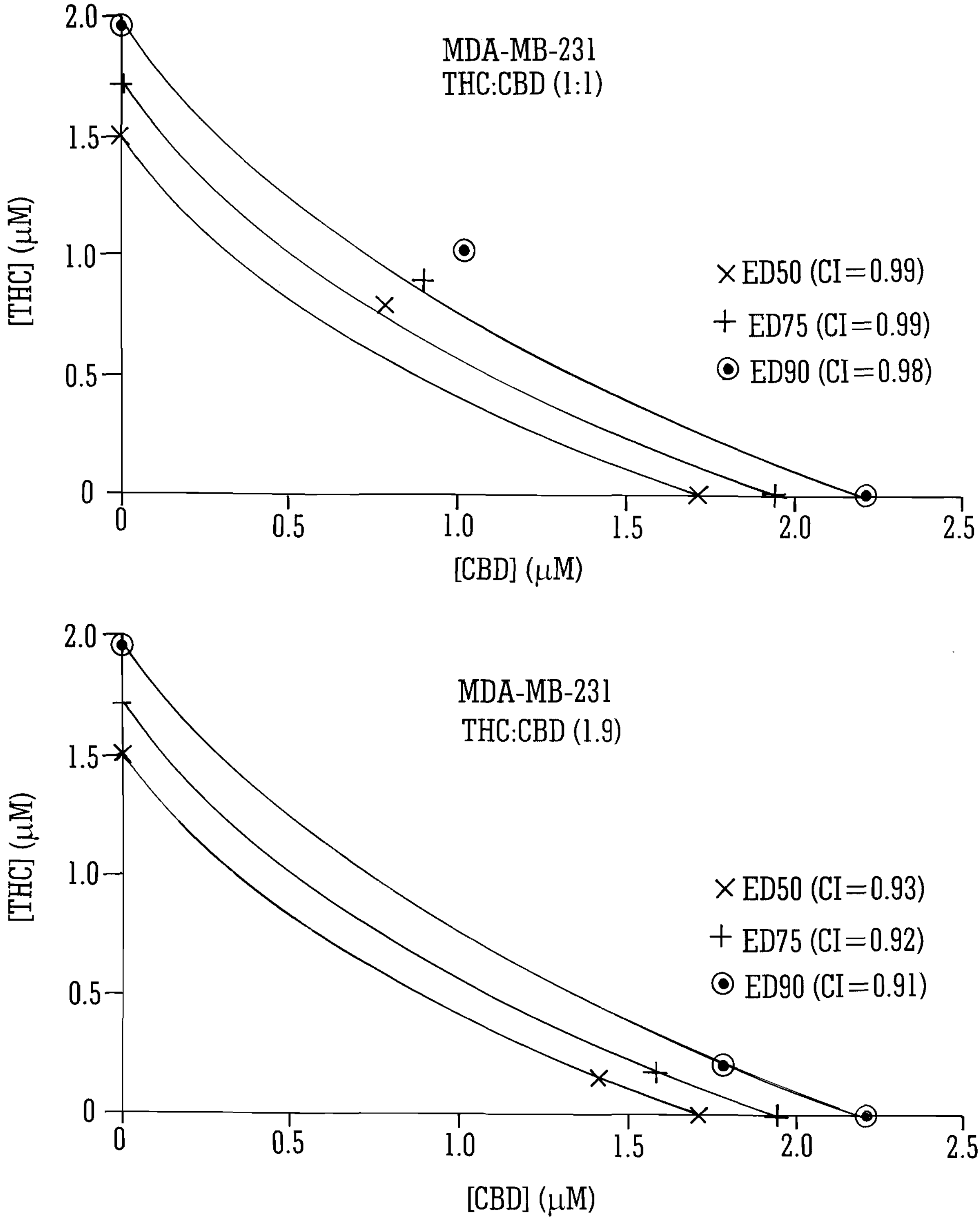
2.0
1.5
1.0
0.5
0
[THC] (μM)
MDA-MB-231
THC:CBD (1:1)
× ED50 (CI=0.99)
+ ED75 (CI=0.99)
⊙ ED90 (CI=0.98)
0
0.5
1.0
1.5
2.0
2.5
[CBD] (μM)
2.0
1.5
1.0
0.5
0
[THC] (μM)
MDA-MB-231
THC:CBD (1.9)
× ED50 (CI=0.93)
+ ED75 (CI=0.92)
⊙ ED90 (CI=0.91)
0
0.5
1.0
1.5
2.0
2.5
[CBD] (μM)

FIG.12

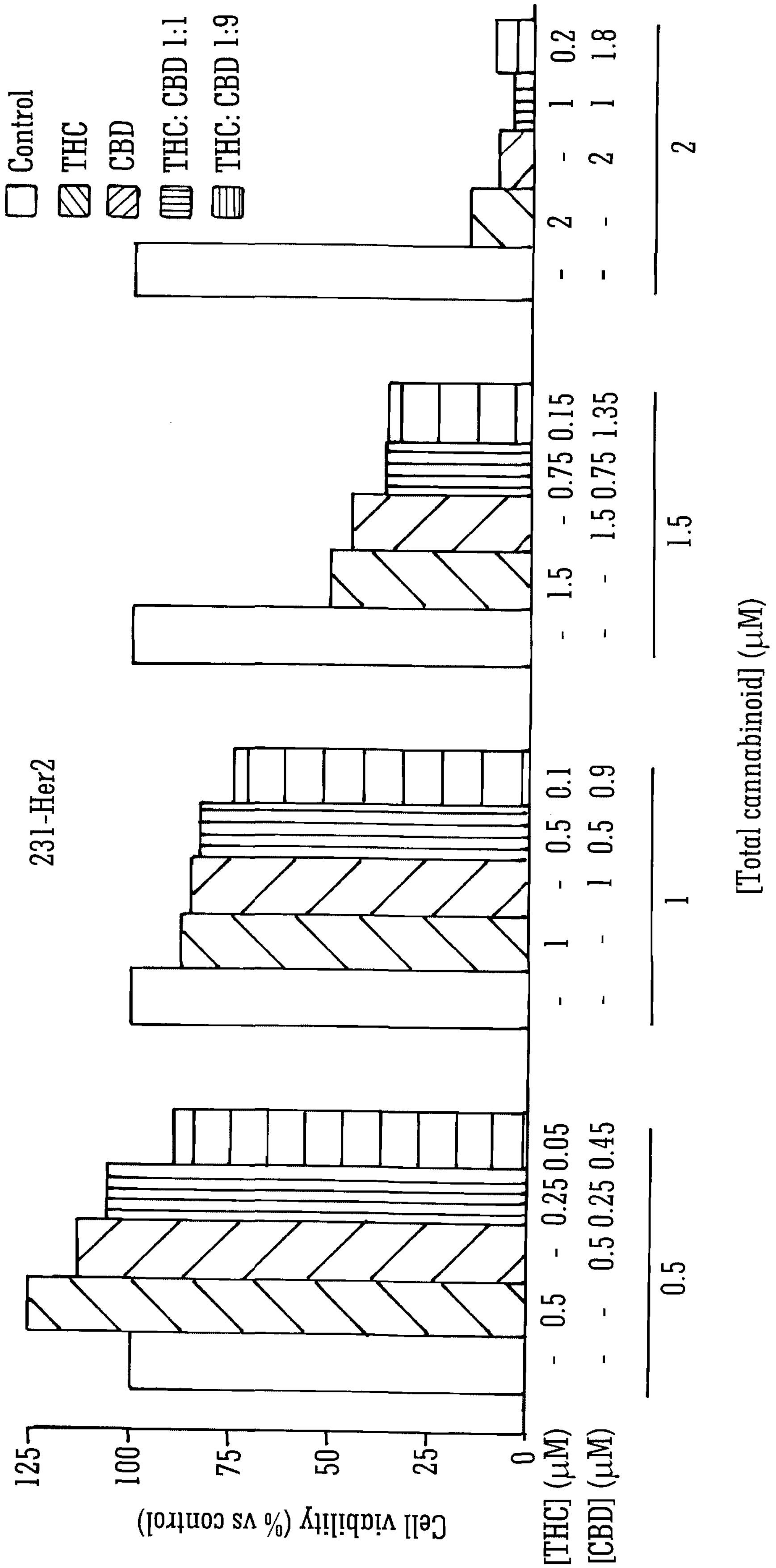
231-Her2
Control
THC
CBD
THC: CBD 1:1
THC: CBD 1:9
Cell viability (% vs control)
125
100
75
50
25
0
[THC] (μM) - 0.5 - 0.25 0.05 - 1 - 0.5 0.1 - 1.5 - 0.75 0.15 - 2 - 1 0.2
[CBD] (μM) - - 0.5 0.25 0.45 - - 1 0.5 0.9 - - 1.5 0.75 1.35 - - 2 1 1.8
0.5
1
1.5
2
[Total cannabinoid] (μM)

FIG.13A

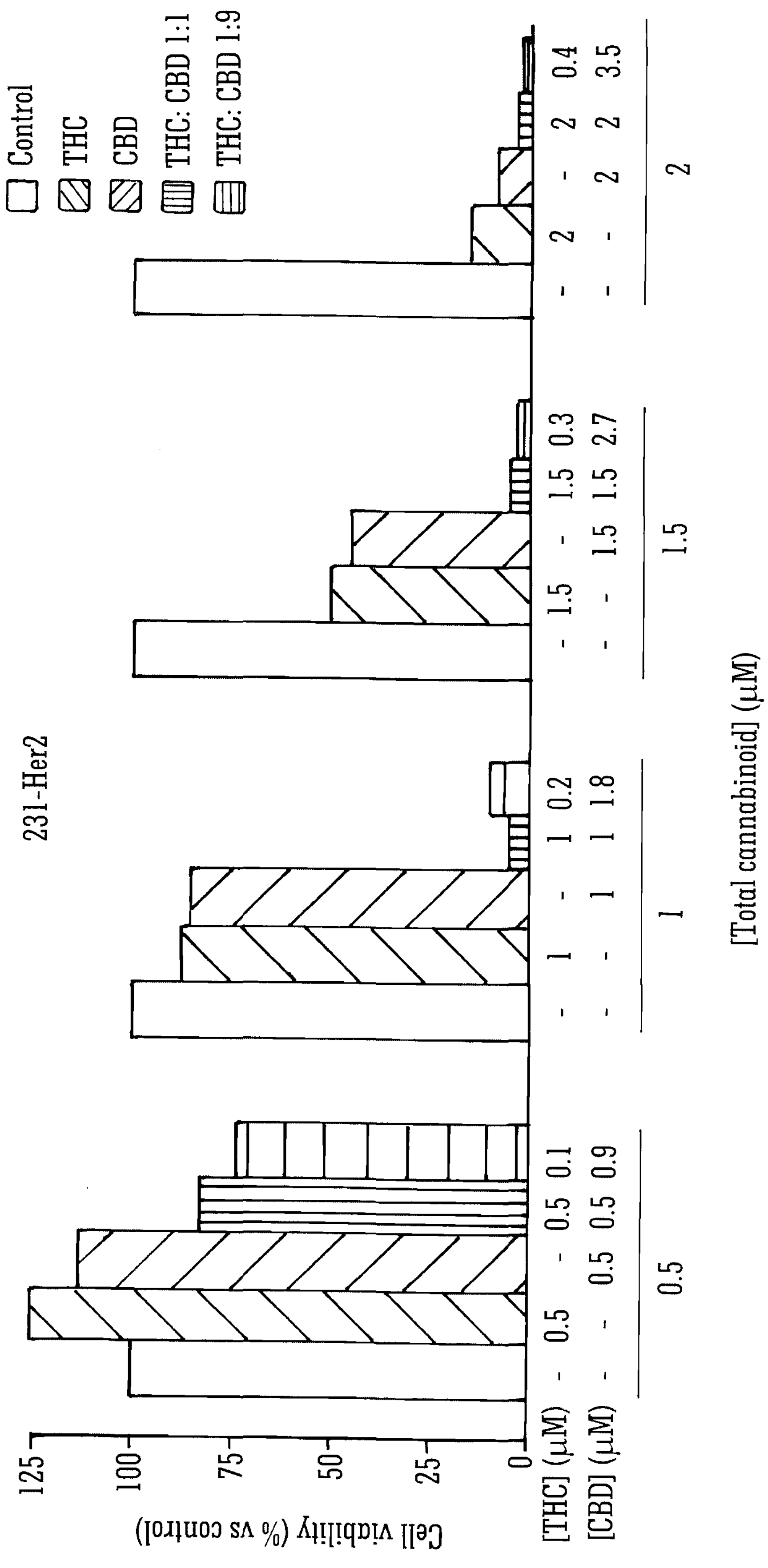
231-Her2
Control
THC
CBD
THC: CBD 1:1
THC: CBD 1:9
Cell viability (% vs control)
125
100
75
50
25
0
[THC] (μM) - 0.5 - 0.5 0.1 - 1 - 1 0.2 - 1.5 - 1.5 0.3 - 2 - 2 0.4
[CBD] (μM) - - 0.5 0.5 0.9 - - 1 1 1.8 - - 1.5 1.5 2.7 - - 2 2 3.5
0.5
1
1.5
2
[Total cannabinoid] (μM)

FIG.13B

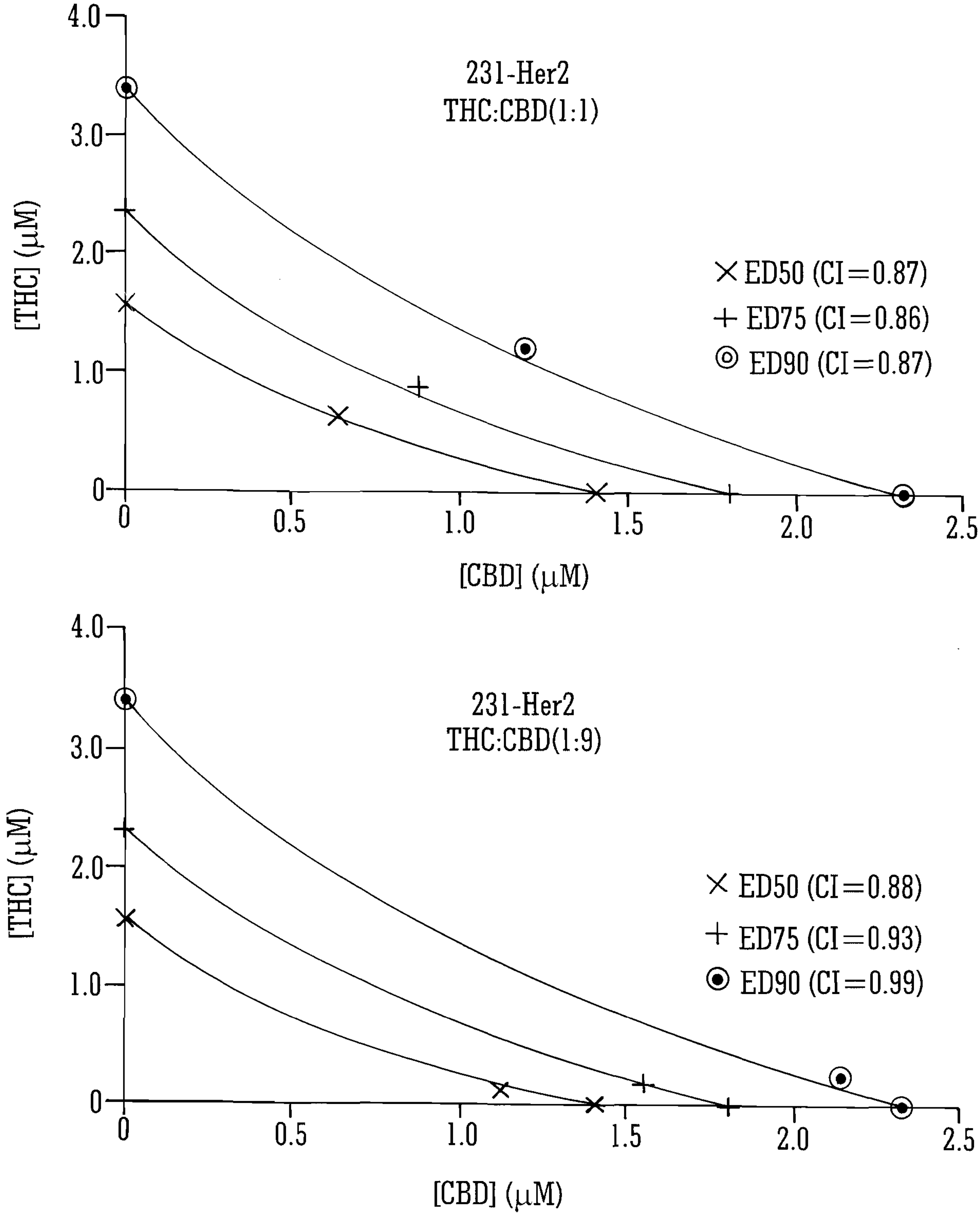
231-Her2
THC:CBD(1:1)
ED50 (CI=0.87)
ED75 (CI=0.86)
ED90 (CI=0.87)
[THC] (μM)
[CBD] (μM)
4.0
3.0
2.0
1.0
0
0
0.5
1.0
1.5
2.0
2.5
231-Her2
THC:CBD(1:9)
ED50 (CI=0.88)
ED75 (CI=0.93)
ED90 (CI=0.99)
[THC] (μM)
[CBD] (μM)

FIG.14

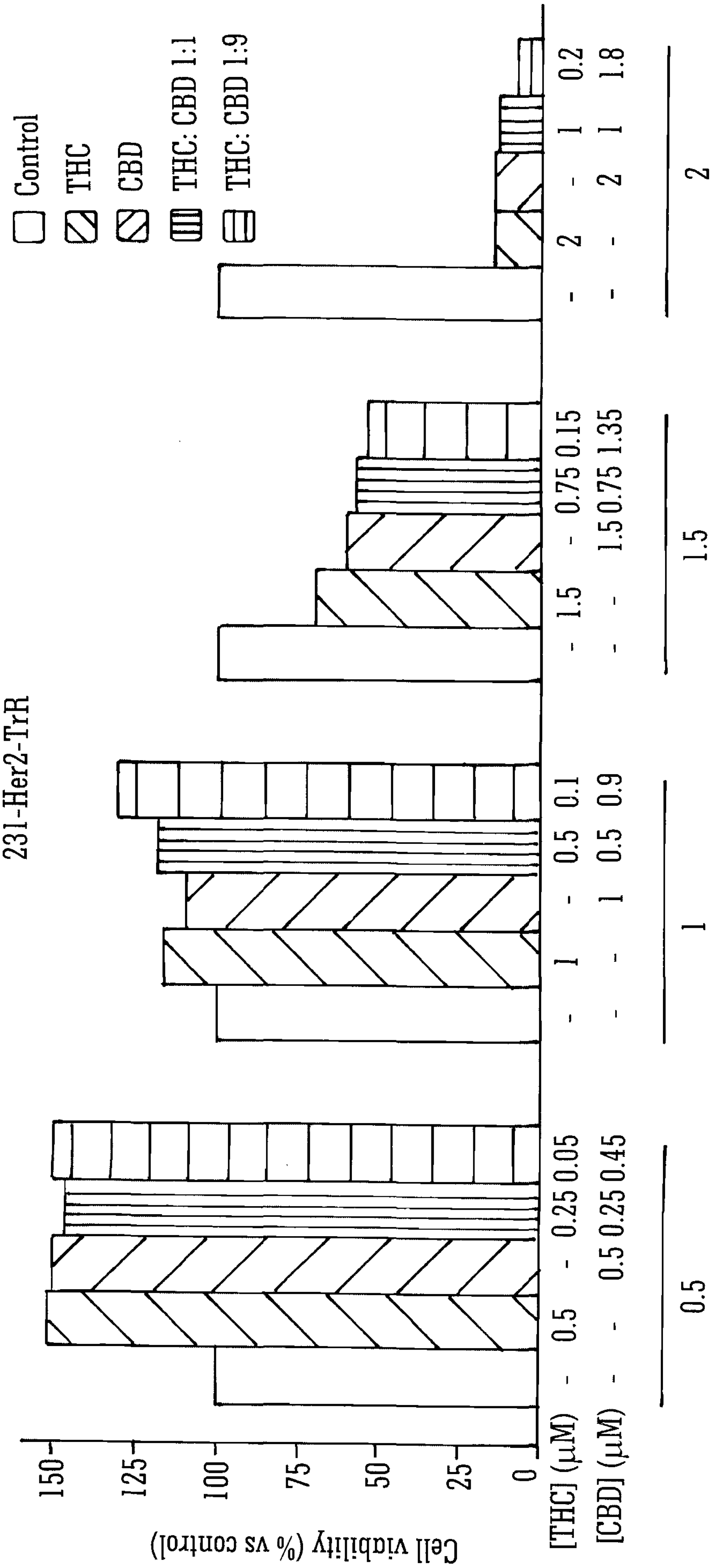
231-Her2-TrR
Control
THC
CBD
THC: CBD 1:1
THC: CBD 1:9
Cell viability (% vs control)
150
125
100
75
50
25
0
[THC] (μM) - 0.5 - 0.25 0.05 - 1 - 0.5 0.1 - 1.5 - 0.75 0.15 - 2 - 1 0.2
[CBD] (μM) - - 0.5 0.25 0.45 - - 1 0.5 0.9 - - 1.5 0.75 1.35 - - 2 1 1.8
0.5
1
1.5
2
[Total cannabinoid] (μM)

FIG.15A

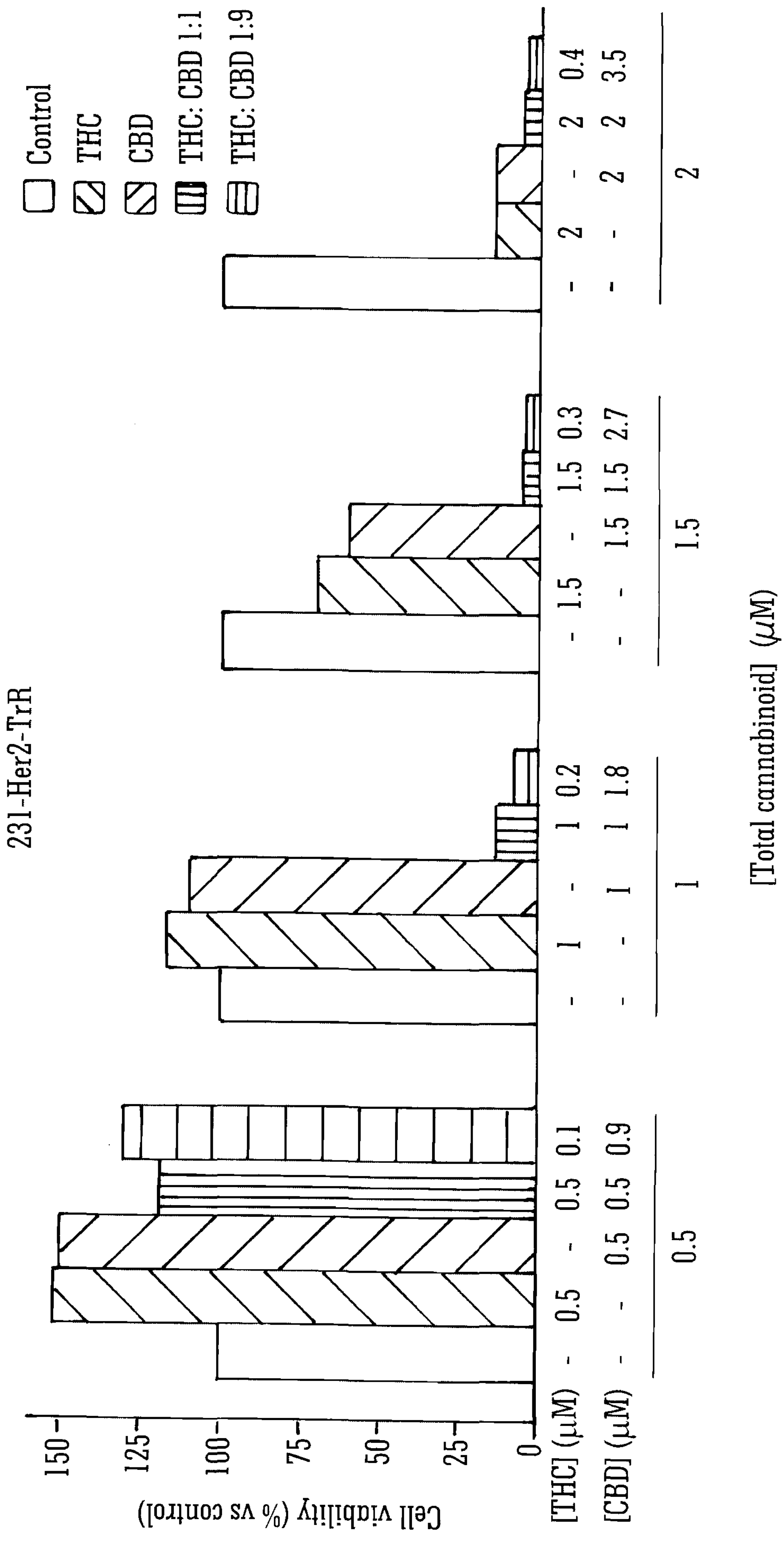
231-Her2-TrR
Control
THC
CBD
THC: CBD 1:1
THC: CBD 1:9
Cell viability (% vs control)
150
125
100
75
50
25
0
[THC] (µM) - 0.5 - 0.5 0.1 - 1 - 1 0.2 - 1.5 - 1.5 0.3 - 2 - 2 0.4
[CBD] (µM) - - 0.5 0.5 0.9 - - 1 1 1.8 - - 1.5 1.5 2.7 - - 2 2 3.5
0.5
1
1.5
2
[Total cannabinoid] (µM)

FIG.15B

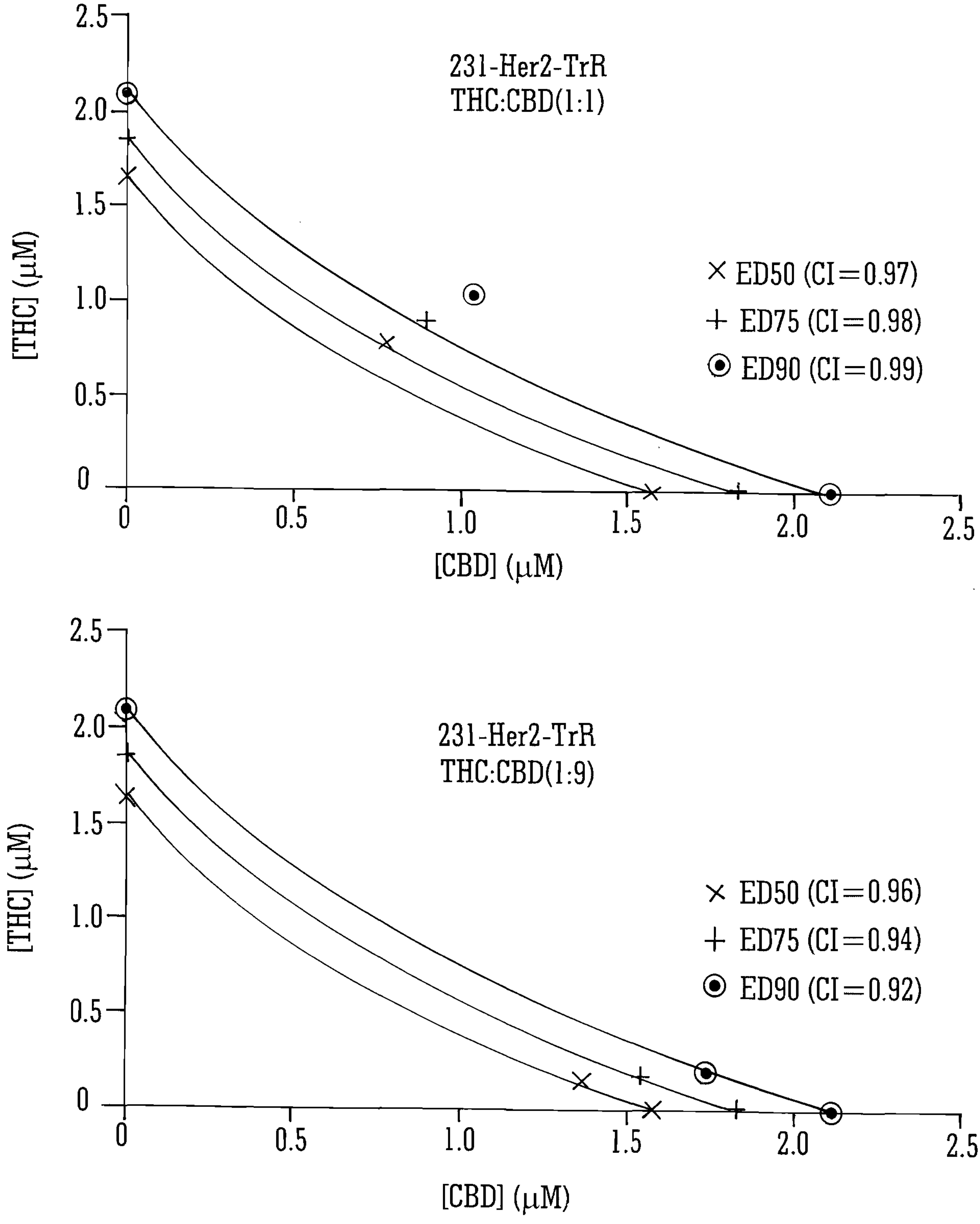
2.5
2.0
1.5
1.0
0.5
0
[THC] (μM)
231-Her2-TrR
THC:CBD(1:1)
× ED50 (CI=0.97)
+ ED75 (CI=0.98)
ED90 (CI=0.99)
0
0.5
1.0
1.5
2.0
2.5
[CBD] (μM)
2.5
2.0
1.5
1.0
0.5
0
[THC] (μM)
231-Her2-TrR
THC:CBD(1:9)
× ED50 (CI=0.96)
+ ED75 (CI=0.94)
ED90 (CI=0.92)
0
0.5
1.0
1.5
2.0
2.5
[CBD] (μM)

FIG.16

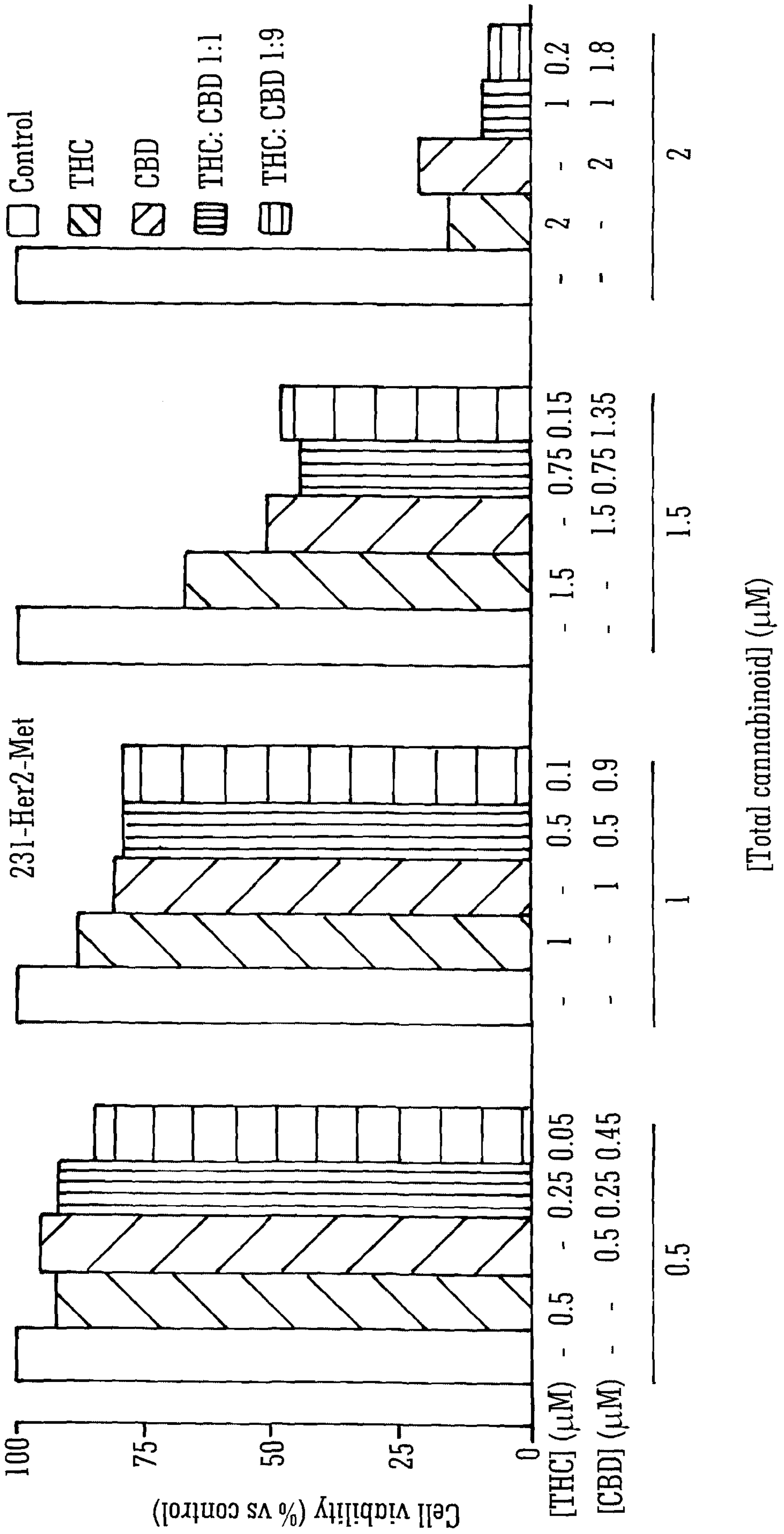
231-Her2-Met
Control
THC
CBD
THC: CBD 1:1
THC: CBD 1:9
Cell viability (% vs control)
100
75
50
25
0
[THC] (μM) - 0.5 - 0.25 0.05 - 1 - 0.5 0.1 - 1.5 - 0.75 0.15 - 2 - 1 0.2
[CBD] (μM) - - 0.5 0.25 0.45 - - 1 0.5 0.9 - - 1.5 0.75 1.35 - - 2 1 1.8
0.5
1
1.5
2
[Total cannabinoid] (μM)

FIG.17A

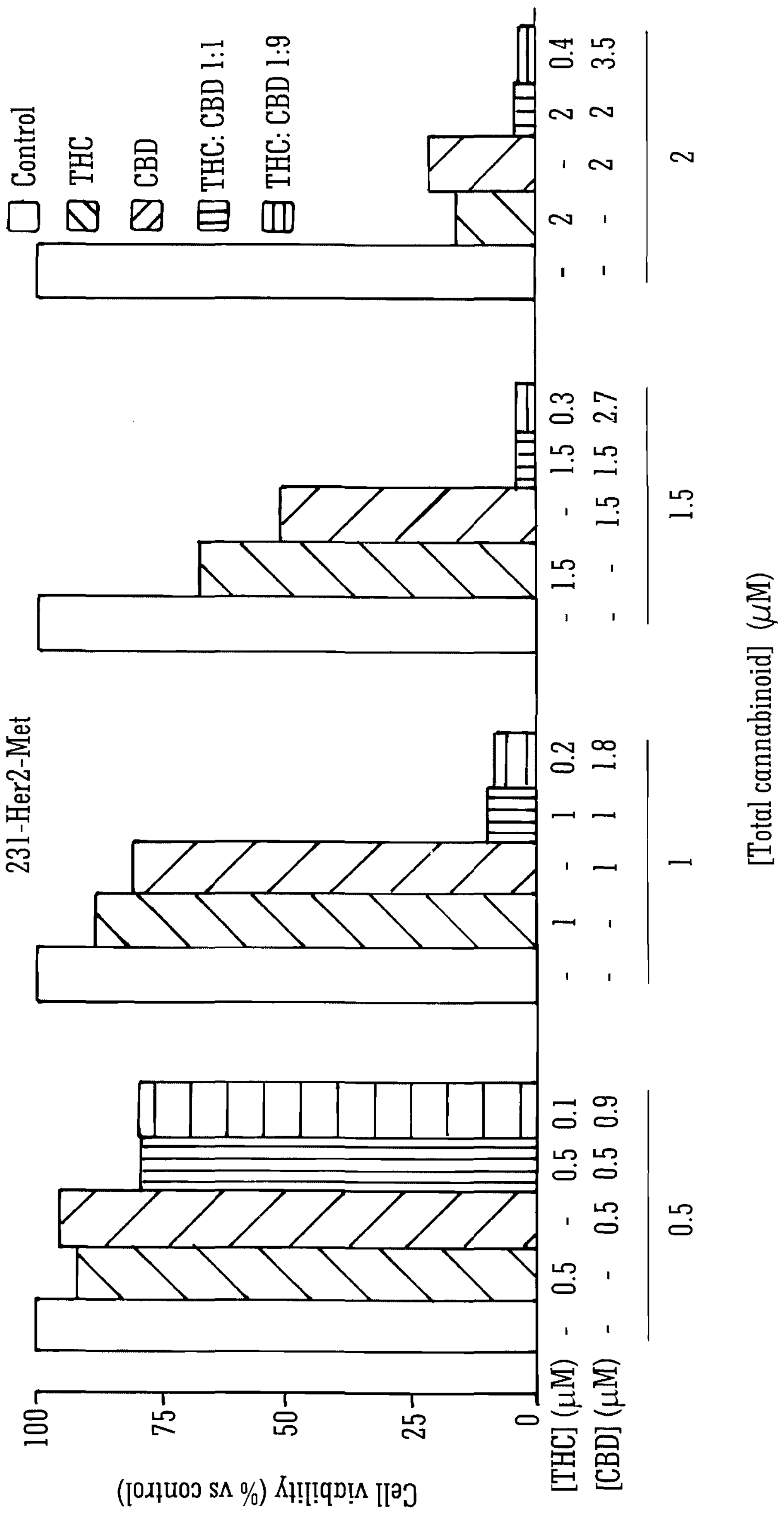
231-Her2-Met
Control
THC
CBD
THC: CBD 1:1
THC: CBD 1:9
Cell viability (% vs control)
100
75
50
25
0
[THC] (μM) - 0.5 - 0.5 0.1 - 1 - 1 0.2 - 1.5 - 1.5 0.3 - 2 - 2 0.4
[CBD] (μM) - - 0.5 0.5 0.9 - - 1 1 1.8 - - 1.5 1.5 2.7 - - 2 2 3.5
0.5
1
1.5
2
[Total cannabinoid] (μM)

FIG.17B

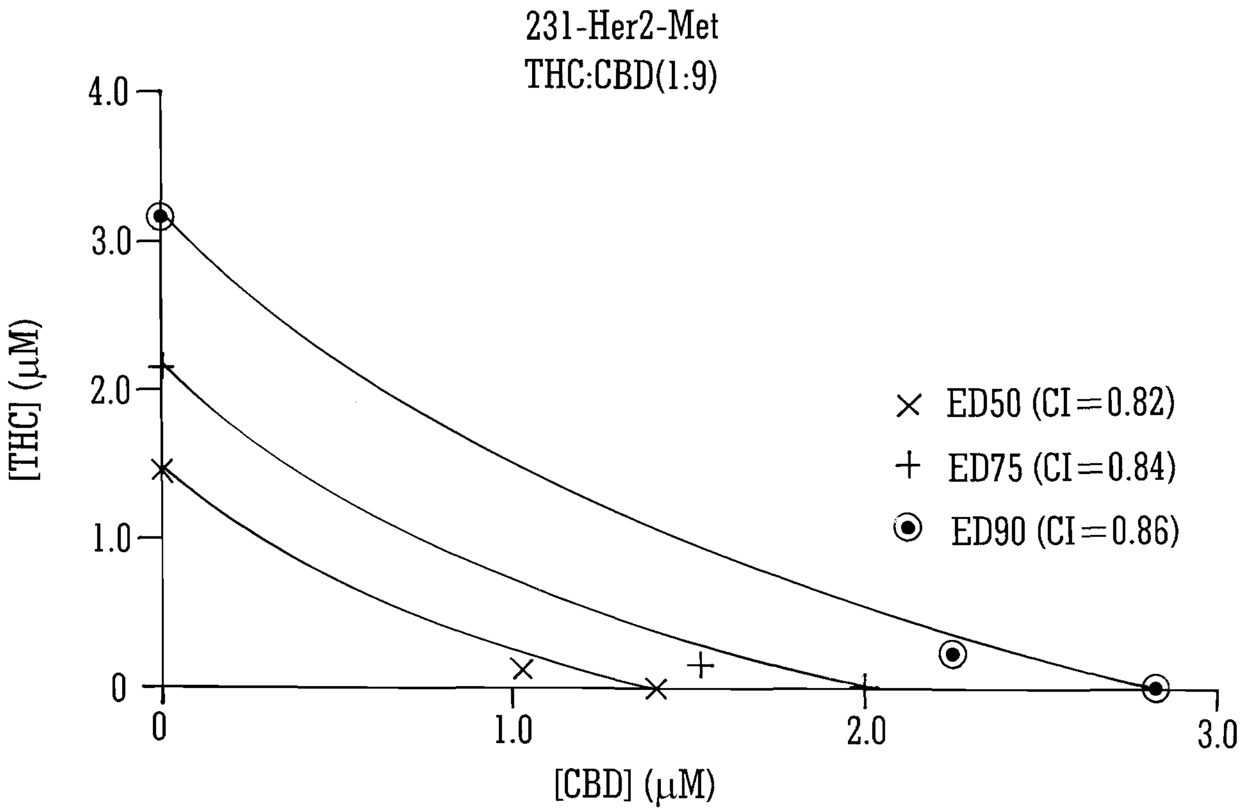
231-Her2-Met
THC:CBD(1:9)
4.0
3.0
2.0
1.0
0
[THC] (μM)
0
1.0
2.0
3.0
[CBD] (μM)
ED50 (CI=0.82)
ED75 (CI=0.84)
ED90 (CI=0.86)

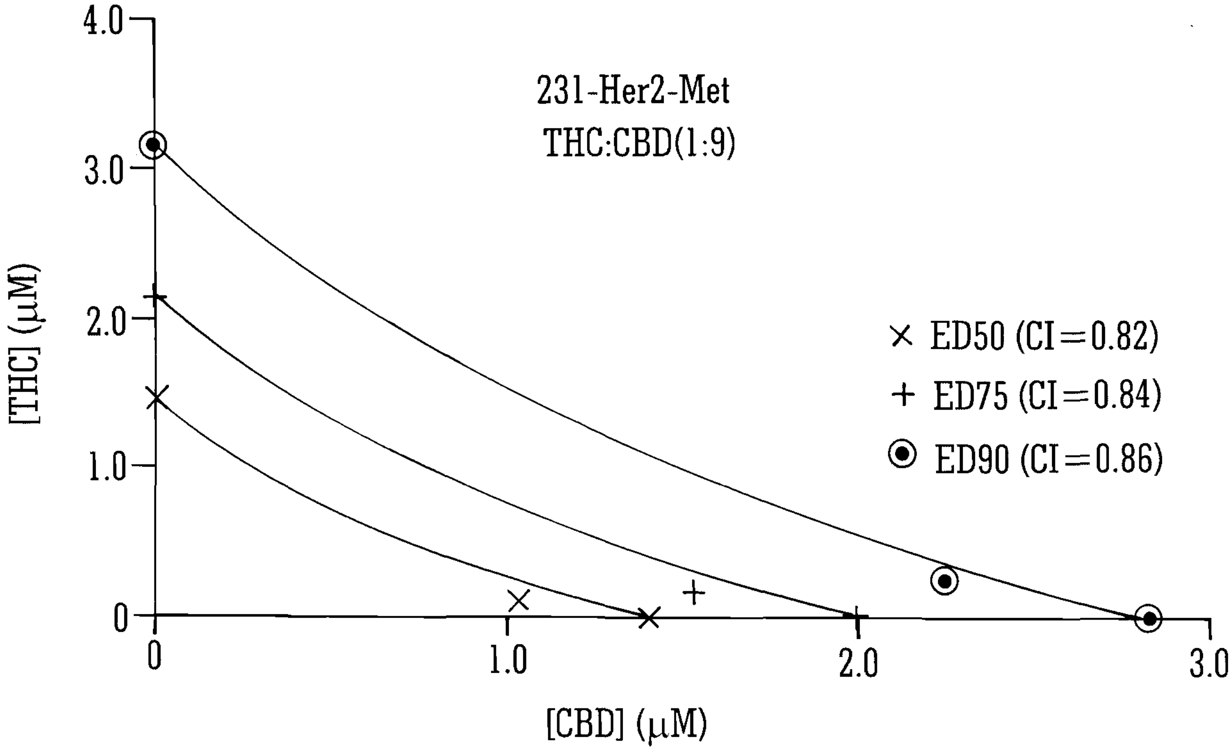
231-Her2-Met
THC:CBD(1:9)
4.0
3.0
2.0
1.0
0
[THC] (μM)
0
1.0
2.0
3.0
[CBD] (μM)
ED50 (CI=0.82)
ED75 (CI=0.84)
ED90 (CI=0.86)

FIG.18

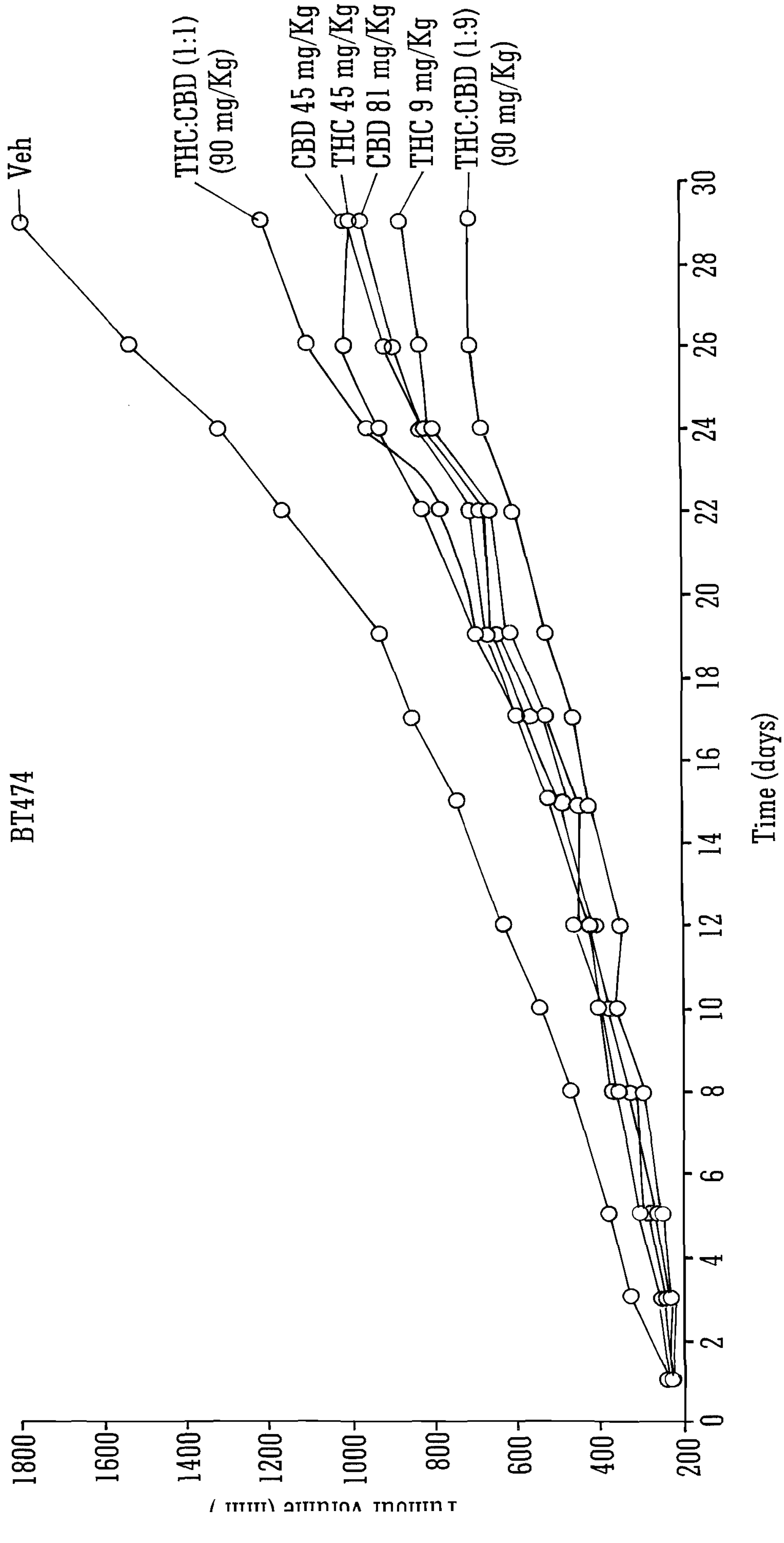
BT474
Veh
THC:CBD (1:1)
(90 mg/Kg)
CBD 45 mg/Kg
THC 45 mg/Kg
CBD 81 mg/Kg
THC 9 mg/Kg
THC:CBD (1:9)
(90 mg/Kg)
Time (days)
0
2
4
6
8
10
12
14
16
18
20
22
24
26
28
30
200
400
600
800
1000
1200
1400
1600
1800

FIG.19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008144475 A
- WO 2009147439 A


### Non-patent literature cited in the description


- Guidance for Industry Botanical Drug Products Draft Guidance. US Department of Health and Human Services, August 2000
- **CAFFAREL, M.M. ; ANDRADAS, C. ; MIRA, E. ; PEREZ-GOMEZ, E. ; CERUTTI, C. ; MORENO-BUENO, G. ; FLORES, J.M. ; GARCIA-REAL, I. ; PALACIOS, J. ; MANES, S. et al.** Cannabinoids reduce ErbB2-driven breast cancer progression through Akt inhibition. *Mol Cancer,* 2010, vol. 9, 196
- **LIGRESTI, A. ; MORIELLO, A.S. ; STAROWICZ, K. ; MATIAS, I. ; PISANTI, S. ; DE PETROCELLIS, L. ; LAEZZA, C. ; PORTELLA, G. ; BIFULCO, M. ; DI MARZO, V.** Antitumor activity of plant cannabinoids with emphasis on the effect of cannabidiol on human breast carcinoma. *J Pharmacol Exp Ther,* 2006, vol. 318, 1375-1387
- **MCALLISTER, S.D. ; CHRISTIAN, R.T. ; HOROWITZ, M.P. ; GARCIA, A. ; DESPREZ, P.Y.** Cannabidiol as a novel inhibitor of Id-1 gene expression in aggressive breast cancer cells. *Mol Cancer Ther,* 2007, vol. 6, 2921-2927
- **MCALLISTER, S.D. ; MURASE, R. ; CHRISTIAN, R.T. ; LAU, D. ; ZIELINSKI, A.J. ; ALLISON, J. ; ALMANZA, C. ; PAKDEL, A. ; LEE, J. ; LIMBAD, C. et al.** Pathways mediating the effects of cannabidiol on the reduction of breast cancer cell proliferation, invasion, and metastasis. *Breast Cancer Res Treat,* 2010